(19) Europäisches Patentamt
European Patent Office
Office européen des brevets

(11) Publication number: 0 452 002 A2

(12) # EUROPEAN PATENT APPLICATION

(21) Application number: 91302763.7

(22) Date of filing: 28.03.91

(51) Int. Cl.[5]: **C07D 495/04**, A01N 43/90, C07D 333/38, C07F 7/10, // (C07D495/04, 333:00, 239:00)

(30) Priority: 30.03.90 US 502342

(43) Date of publication of application: 16.10.91 Bulletin 91/42

(84) Designated Contracting States: AT BE CH DE DK ES FR GB GR IT LI LU NL SE

(71) Applicant: DOWELANCO
9002 Purdue Road
Indianapolis, Indiana 46268 (US)

(72) Inventor: Edie, Ronnie Gerald
123 North Windswept Road
Greenfield, Indiana 46140 (US)
Inventor: Hackler, Ronald Ervin
5234 Hawks Point Road
Indianapolis, Indiana 46226 (US)
Inventor: Krumkains, Eriks Viktors
6333 North Rural
Indianapolis, Indiana 46220 (US)

(74) Representative: Raynor, John et al
W.H. Beck, Greener & Co 7 Stone Buildings
Lincoln's Inn
London WC2A 3SZ (GB)

(54) **Thienopyrimidine derivatives.**

(57) 4-Substituted-thieno[2,3-d], [3,2-d], and [3,4-d]pyrimidines having fungicidal, insecticidal, and miticidal utility are disclosed. Related 3-substituted-thieno[2,3-d], [3,2-d], and [3,4-d]pyrimidin-4-(3H)-imines are useful as fungicides, insecticides, and miticides, and also as intermediates in preparing, N-substituted-thienopyrimidin-4-amines. Related o-cyano-amidines are useful as intermediates in preparing the 3-substituted-thieno[2,3-d], [3,2-d], and [3,4-d]pyrimidin–(3H)-imines.

EP 0 452 002 A2

Field of the Invention

This invention provides new compounds that have excellent plant fungicide activity. Some of the compounds have also demonstrated insecticidal and miticidal activity. The invention also provides compositions and combination products that contain a compound of the invention as active ingredient, as well as providing fungicidal, miticidal, and insecticidal methods.

There is an acute need for new fungicides, insecticides, and miticides, because target pathogens are rapidly developing resistance to currently used pesticides. Widespread failure of N-substituted azole fungicides to control barley mildew was observed in 1983, and has been attributed to the development of resistance. At least 50 species of fungi have developed resistance to the benzimidazole fungicides. The field performance of DMI (demethylation inhibitor) fungicides, which are now widely relied on to protect cereal crops from powdery mildew, has declined since they were introduced in the 1970's. Even recently introduced fungicides, like the acylalanines, which initially exhibited excellent control of potato late blight and grape downy mildew in the field, have become less effective because of widespread resistance. Similarly, mites and insects are developing resistance to the miticides and insecticides in current use. Resistance to insecticides in arthropods is widespread, with at least 400 species resistant to one or more insecticides. The development of resistance to some of the older insecticides, such as DDT, the carbamates, and the organophosphates, is well known. But resistance has even developed to some of the newer pyrethroid insecticides and miticides. Therefore a need exists for new fungicides, insecticides, and miticides.

Summary of the Invention

This invention provides compounds of the formula (1):

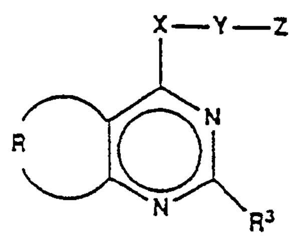

(1)

wherein

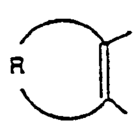

is

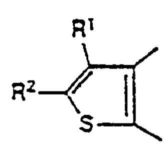

,

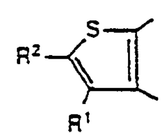

, or

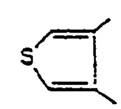

$R^1$ is H, $(C_1$-$C_4)$ alkyl, or phenyl optionally substituted with halo, $(C_1$-$C_4)$ alkyl, $(C_1$-$C_4)$ alkoxy, halo $(C_1$-$C_4)$ alkyl, or halo $(C_1$-$C_4)$ alkoxy;

$R^2$ is H, $C_1$-$C_4$ alkyl, nitro, halo; or

$R^1$ and $R^2$ combine to form $-(CH_2)_4-$;

$R^3$ is H, $C_1$-$C_4$ alkyl, halo $(C_1$-$C_4)$ alkyl, phenyl, or substituted phenyl;

-XYZ together form 1H-1,2,4-triazol-1-yl; or

X is O, S, $NR^4$, or $CR^5R^6$, where $R^4$ is H, $(C_1$-$C_4)$ alkyl, or $(C_1$-$C_4)$ acyl, and $R^5$ and $R^6$ are independently H, $(C_1$-$C_4)$ alkyl, $(C_2$-$C_4)$ alkenyl or -alkynyl, CN, or OH, or $R^5$ and $R^6$ combine to form a carbocyclic ring containing four to six carbon atoms; and

-YZ together form a $C_5$-$C_{12}$ saturated or unsaturated hydrocarbon chain, straight chain or branched; optionally including a hetero atom selected from O, $NR^4$, S, SO, $SO_2$, or $SiR^7R^8$, where $R^4$ is as defined above and $R^7$ and $R^8$ are independently $(C_1$-$C_4)$ alkyl, $(C_3$-$C_4)$ branched alkyl, phenyl, or substituted phenyl, and optionally substituted with $(C_1$-$C_4)$ alkyl, $(C_2$-$C_4)$ alkenyl or -alkynyl, branched $(C_3$-$C_7)$ alkyl, $(C_3$-$C_7)$ cycloalkyl or -cycloalkenyl, halo, halo $(C_1$-$C_4)$ alkyl, halo $(C_1$-$C_4)$ alkoxy, hydroxy, or $(C_1$-$C_4)$ acyl; or

Y is a bond or a bivalent hydrocarbon radical one to six carbon atoms long, optionally including a carbocyclic ring, and optionally including a hetero atom selected from O, $NR^4$, S, SO, $SO_2$, or $SiR^7R^8$, where $R^4$, $R^7$, and

$R^8$, are as defined above, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl; and

Z is

(a) ($C_3$-$C_8$) cycloalkyl or cycloalkenyl, optionally substituted with ($C_1$-$C_4$) alkyl, ($C_1$-$C_4$) alkoxy, halo ($C_1$-$C_4$) alkyl, halo ($C_1$-$C_4$) alkoxy, halo, hydroxy, or ($C_1$-$C_4$) acyl;

(b) a phenyl group of the formula (2)

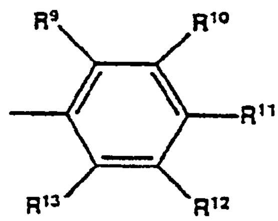

(2)

where

$R^9$ to $R^{13}$ are independently

H,

halo,

I,

($C_3$-$C_8$) cycloalkyl or -cycloalkenyl,

phenoxy, or

substituted phenoxy,

phenylthio, or substituted phenylthio,

phenyl, or substituted phenyl,

$NO_2$,

acetoxy,

OH,

CN,

$SiR^7R^8R^{14}$ or $OSiR^7R^8R^{14}$, where $R^7$, and $R^8$ are as defined above and $R^{14}$ is ($C_1$-$C_4$) alkyl, ($C_3$-$C_4$) branched alkyl, phenyl, or substituted phenyl,

$NR^{15}R^{16}$, where $R^{15}$ and $R^{16}$ are independently H, ($C_1$-$C_4$) alkyl, or ($C_1$-$C_4$) acyl,

$S(O)R^{17}$, or $SO_2R^{17}$, where $R^{17}$ is ($C_1$-$C_{10}$) alkyl, phenyl, or substituted phenyl;

a $C_1$-$C_{12}$ saturated or unsaturated hydrocarbon chain, straight chain or branched optionally including a hetero atom selected from O, S, SO, $SO_2$, $NR^4$, or $SiR^7R^8$, where $R^4$, $R^7$, and $R^8$ are as defined above, and optionally substituted with halo, halo ($C_1$-$C_4$) alkoxy, hydroxy, ($C_3$-$C_8$) cycloalkyl or cycloalkenyl, ($C_1$-$C_4$) acyl, phenoxy, substituted phenoxy, phenyl, substituted phenyl, phenylthio, or substituted phenylthio;

($C_1$-$C_7$) alkoxy optionally substituted with halo, phenyl, substituted phenyl, ($C_3$-$C_8$) cycloalkyl or cycloalkenyl, phenoxy, or substituted phenoxy;

($C_1$-$C_7$) alkylthio optionally substituted with halo, phenyl, substituted phenyl, ($C_3$-$C_8$) cycloalkyl or cycloalkenyl, phenoxy or substituted phenoxy; or

$R^{10}$ and $R^{11}$ combine to form $-O-CF_2-O-$; (c) a furyl group of formula (3)

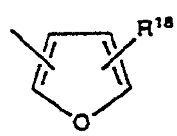

(3)

where $R^{18}$ is H, halo, halomethyl, CN, $NO_2$, ($C_1$-$C_4$) alkyl, ($C_3$-$C_4$) branched alkyl, phenyl, ($C_1$-$C_4$) alkoxy;

(d) a thienyl group of the formula (4)

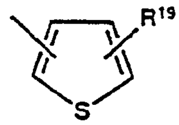

(4)

where $R^{19}$ is $R^{18}$ as defined in paragraph (c) or thienyl;

(e) a group of formula (5) or (6)

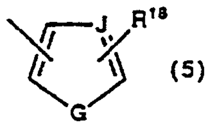

(5)

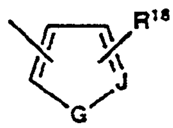

(6)

where $R^{18}$ is as defined in paragraph (c), J is N or CH, and G is O, $NR^{20}$, or S, provided that if J is not N then G is $NR^{20}$, where $R^{20}$ is H, $(C_1-C_4)$ alkyl, $(C_1-C_4)$ acyl, phenylsulfonyl, or substituted phenylsulfonyl;

(f) a group selected from

optionally substituted naphthyl, dihydronaphthyl, tetrahydronaphthyl, and decahydronaphthyl;
optionally substituted pyridyl;
optionally substituted indolyl;
1,3-benzodioxolyl;
2,6-dimethyl-4-morpholinyl; and
1-adamantyl;

or an acid addition salt of a compound of formula (1); provided that:

(1) if the compound of formula (1) is a thieno[2,3-d]pyrimidin-4-amine, then Y does not include a heteroatom and is at least two carbon atoms long, and Z is 2-naphthyl or a phenyl group of formula (2) wherein at least one of $R^9$ to $R^{13}$ is phenyl, substituted phenyl, phenoxy, substituted phenoxy, phenylthio, substituted phenylthio, $(C_3-C_4)$ branched alkyl, $(C_1-C_4)$ alkoxy, halo, halo $(C_1-C_4)$ alkoxy, or halo $(C_1-C_4)$ alkyl;

(2) if the compound of formula (1) is a thieno[2,3-d]pyrimidine wherein X is O, then YZ is neither unsubstituted phenyl nor benzyl; and

(3) if the compound of formula (1) is a 7-methylthieno[3,2-d]pyrimidine, then -XYZ is not N-[2-(2-pyridyl)ethyl]amino, and X-Y does not include the group -NH-N=CH-.

Compounds of formula (1) are useful as fungicides, insecticides, and miticides.

The invention also provides novel compounds of the formula

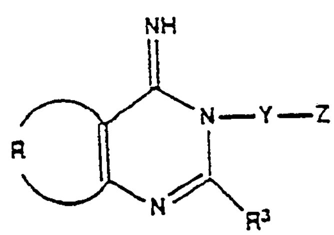

(7)

where

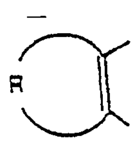

,

$R^3$, Y, and Z are as defined in formula (1). Compounds of formula (7) are useful as fungicides, is some cases as insecticides, and also as intermediates in preparation of compounds of formula (1).

The invention also provides novel compounds of the formula (8)

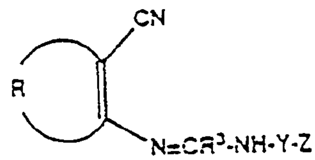

(8)

where

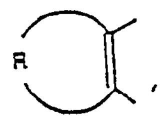

$R^3$, Y, ans Z are as defined in formula (1). Compounds of formula (8) are unisolated intemediates in preparation of compounds of formula (1) and (7).

The invention includes with it scope compositions for fungicidal, insecticidal or miticidal use, comprising a compound of formula (1) or (7) in combination with a phytologically-acceptable carrier. Such compositions may optionally contain additional active ingredients, such as an additional fungicidal, miticidal, or insecticidal ingredient.

Such compositions preferably comprise at least 1% by weight of a compound of formula (1) or (7).

The insecticide and miticide compositions of the invention comprise an insect- or mite-inactivating amount of a compound of formula (1) or (7) in combination with a carrier. Such compositions may optionally contain additional active ingredients, such as an additional fungicidal, miticidal, or insecticidal ingredient.

The invention also provides a method of the use of a compound of formula (1) or (7) as a fungicide, an insecticide, or a miticide.

Throughout this document, all temperatures are given in degrees Celsius, and all percentages are weight percentages unless otherwise stated.

The term "halo" refers to a F, Cl, or Br atom.

The tem "($C_1$-$C_7$) alkoxy" refers to straight or branched chain alkoxy groups.

The term "($C_1$-$C_7$) alkylthio" refers to straight and branched chain alkylthio groups.

The term "halo ($C_1$-$C_7$) alkyl" refers to a ($C_1$-$C_7$) alkyl group, straight chain or branched, substituted with one or more halo atoms.

The tem "halo ($C_1$-$C_7$) alkoxy" refers to a ($C_1$-$C_7$) alkoxy group substituted with one or more halo groups.

The term "halo ($C_1$-$C_4$) alkylthio" refers to a ($C_1$-$C_4$) alkylthio group, straight chain or branched, substituted with one or more halo atoms.

The term "substituted phenyl" refers to phenyl substituted with up to three groups selected from halo, I, ($C_1$-$C_{10}$) alkyl, branched ($C_3$-$C_6$) alkyl, halo ($C_1$-$C_4$) alkyl, hydroxy ($C_1$-$C_4$) alkyl, ($C_1$-$C_4$) alkoxy, halo ($C_1$-$C_4$) alkoxy, phenoxy, substituted phenoxy, phenyl, substituted phenyl, $NO_2$, OH, CN, ($C_1$-$C_4$) alkanoyloxy, or benzyloxy.

The terms "substituted naphtynyl", "substituted pyridyl" and "substituted indolyl" refer to these ring systems substituted with halo, halo ($C_1$-$C_4$) alkyl, CN, $NO_2$, ($C_1$-$C_4$) alkyl, ($C_3$-$C_4$) branched alkyl, phenyl, ($C_1$-$C_4$) alkoxy, or halo ($C_1$-$C_4$) alkoxy.

The term "substituted phenoxy" refers to phenoxy substituted with up to three groups selected from halo, I, ($C_1$-$C_{10}$) alkyl, branched ($C_3$-$C_6$) alkyl, halo ($C_1$-$C_7$) alkyl, hydroxy ($C_1$-$C_7$) alkyl, ($C_1$-$C_7$) alkoxy, halo ($C_1$-$C_7$) alkoxy, phenoxy, substituted phenoxy, phenyl, substituted phenyl, $NO_2$, OH, CN, ($C_1$-$C_4$) alkanoyloxy, or benzyloxy.

The term "carbocyclic ring" refers to a saturated or unsaturated carbocyclic ring containing three to seven carbon atoms.

The terms "substituted phenylthio" and "substituted phenyl sulfonyl" refer to such groups substituted with up to three groups selected from halo, I, ($C_1$-$C_{10}$) alkyl, branched ($C_3$-$C_6$) alkyl, halo ($C_1$-$C_7$) alkyl, hydroxy ($C_1$-$C_7$) alkyl, ($C_1$-$C_7$) alkoxy, halo ($C_1$-$C_7$) alkoxy, phenoxy, substituted phenoxy, phenyl, substituted phenyl, $NO_2$, OH, CN, ($C_1$-$C_4$) alkanoyloxy, or benzyloxy.

The term "unsaturated hydrocarbon chain" refers to a hydrocarbon chain containing one or more sites of unsaturation.

The term "HPLC" refers to a high pressure liquid chromatography.

The term "bivalent hydrocarbon radical" refers to bivalent radicals derived from normal alkanes by removal

of hydrogen atoms from each of the two terminal carbon atoms of the chain, e.g. methylene, ethylene, tri-methylene, tetramethylene, etc.

Compounds

Compounds of formula (1) include thieno(2,3-d)-pyrimidines of formula (1a):

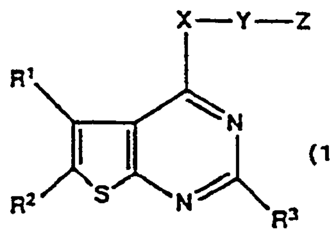

(1a)

thieno(3,2-d)pyrimidines of formula (1b)

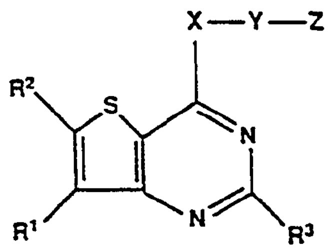

(1b)

and thieno(3,4-d)pyrimidines of formula (1c)

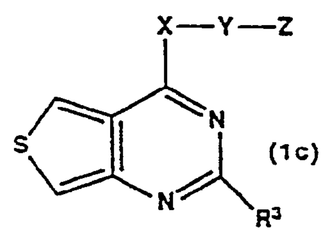

(1c)

While all of the compounds of the invention are useful fungicides, certain classes are preferred for reasons of greater efficacy or ease of synthesis, viz:

a) thieno[2,3-d]pyrimidines of formula (1a);
b) 5-methylthieno[2,3-d]pyrimidines of formula (1a);
c) compounds of formula (1) wherein Y is $-(CH_2)_2-$;
d) compounds of formula (1) wherein X is NH;
e) compounds of formula (1) wherein Z is 3-fluoro-4-methoxyphenyl;
f) compounds of formula (1) wherein Z is 4-chlorophenyl;
g) compounds of formula (1) wherein Z is 3-(trifluoromethyl)phenyl;
h) compounds of formula (1) wherein Z is phenyl;
i) compounds of formula (1) wherein Z is 4-(1,1,2,2-tetrafluoroethoxy)phenyl;
j) compounds of formula (1) wherein Z is 4(methoxy)phenyl;
k) compounds of formula (1) wherein Z is 2-naphthyl;
l) compounds of formula (1) wherein Z is 4(t-butyl)phenyl;
m) compounds of formula (1) wherein Z is 4-(i-propyl)phenyl;
n) compounds of formula (1) wherein Z is 4-(ethoxy)phenyl;
o) compounds of formula (1) wherein Z is 3(methoxy)phenyl;
p) compounds of formula (1) wherein Z is 4-(2,2,2-trifluoroethoxy)phenyl.
q) compounds of formula (1) wherein Z is (haloalkoxy)phenyl.
r) compounds of formula (1) wherein Z is (haloalkyl)phenyl.

s) compounds of formula (1) wherein Z is 3,4-(difluoromethylenedioxy)phenyl.

Synthesis

The compounds of this invention are made using well known chemical procedures. The required starting materials are commercially available, or they are readily synthesized using standard procedures.

Synthesis of Compounds Wherein X is $NR^4$

Compounds of formula (1) wherein X is NH are represented by the following formula (1d):

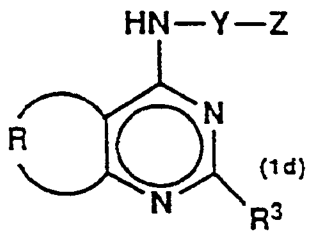

(1d)

wherein each of the variable substitutents are as defined above for formula (1). Compounds of formula (1d) wherein $R^3$ is H can be prepared by the procedure shown in the following Scheme 1:

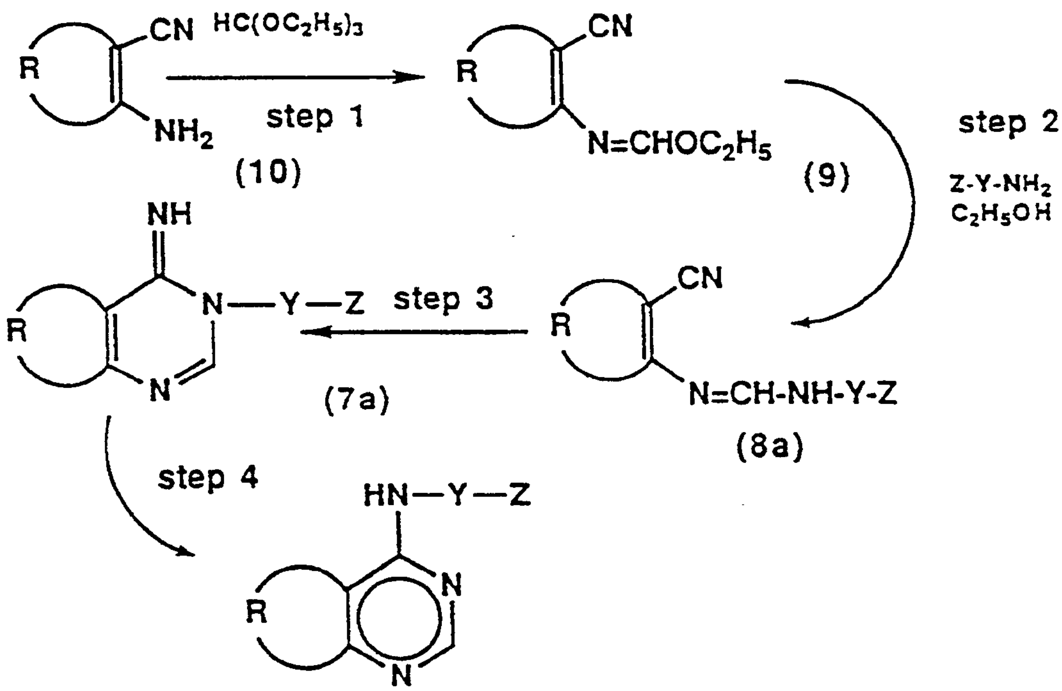


(10) (9) (8a) (7a)

Scheme 1 represents an application of the general procedure that is described in Edward C. Taylor and Alexander McKillop, The Chemistry of Cyclic Enaminonitriles and o-Aminonitriles (Interscience 1970); US Patent No. 4,562,193; J.A.C.S. 90, 2661 (1968); and J. Org. Chem. 32, 2376 (1967).

In step 1 of Scheme 1 an o-aminonitrile (10) is condensed with an orthoester of the formula $HC(OC_2H_5)_3$ or $HC(OCH_3)_3$. The reaction is optionally carried out in the presence of acetic anhydride.

In step 2 of Scheme 1, the imino ether (9) produced in step 1 is reacted with a primary amine of the formula $NH_2$-Y-Z, where Y and Z are as defined in formula (1). The preferred solvent is ethyl alcohol. If step 2 is carried out at room temperature, the novel o-cyano-amidine of formula (8a) can be isolated. At elevated temperatures cyclization of the o-cyanoamidine (step 3) proceeds, delivering the imine of formula (7a).

In Step 4, Dimroth rearrangement of the imine of formula (7a) provides the N-substituted-4-amine of formula (1d). Rearrangement is favored by basic conditions or elevated temperatures.

o-Aminonitriles of formula (10), used as starting materials in Scheme 1, are prepared using known procedures, e.g., those described in Acta Chemica Scandinavica B29 224-232 (1975) (3-amino-2-cyanothiophene); Berichte 99, 94-100 (1966) (2-amino-3-cyanothiophene). Berichte 98, 3571-3577 (1965) (2-amino-3-cyanothiophenes); U.S. Patent No. 4,240,820 (2-amino-3-cyanothiophenes);

Compounds of formula (1d) wherein $R^3$ is other than H can be prepared by the general procedure described in Chemica Scripta 24, 208-223 (1984); Chemica Scripta 28, 195-200 (1988); Liebigs Ann. Chem. 2066 (1983); Chemica Scripta 23, 73 (1984); and Chemica Scripta 18, 245 (1981), as illustrated in Scheme 2.

Scheme 2

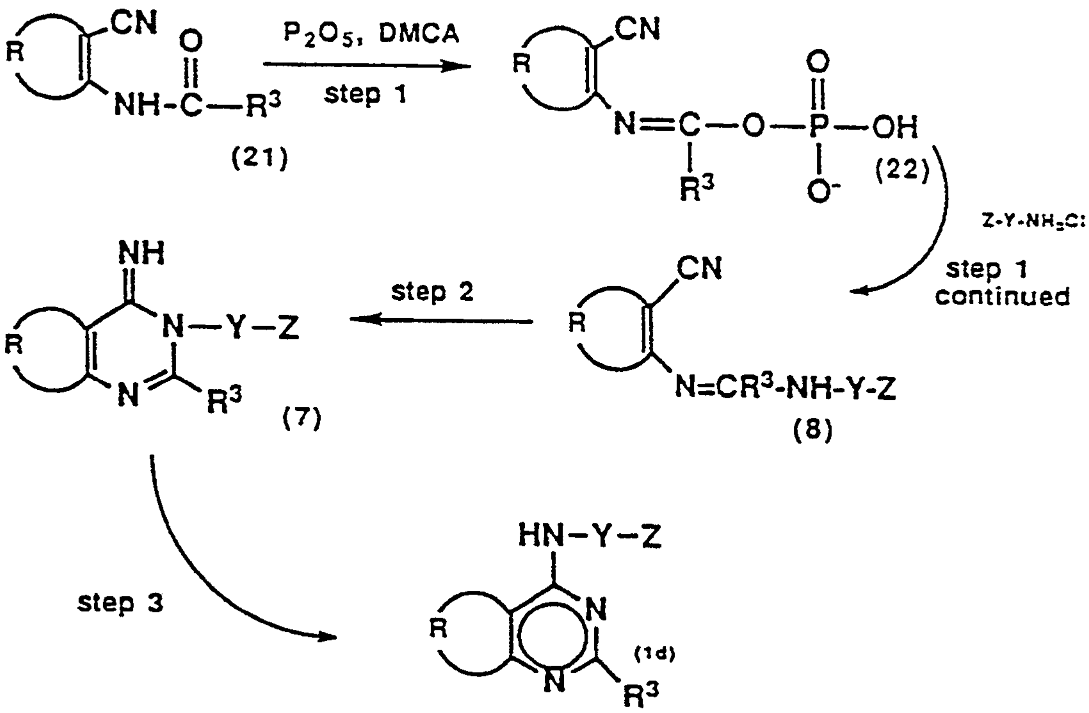


In step 1 of Scheme 2, the o-(acylamino)nitrile of formula (21) is reacted with phosphorus pentoxide, a tertiary amine such as triethylamine or N,N-dimethylcyclohexylamine (DMCA), and an amine hydrochloride of the formula Z-Y-$NH_3$·HCl to give the amidine intermediate of formula (8), which can cyclize, as in Scheme 1 to give the imino compound of formula (7). Upon heating, particularly in the presence of base, the imino compounds rearrange to provide the N-substituted-4-amino compounds of formula (1d) (step 3).

Compounds of formula (1) wherein X is $NR^4$ and $R^4$ is H or $C_1$-$C_4$ allkyl may also be prepared by aminating a suitably substituted intermediate of the formula (23)

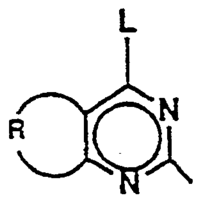
(23)

where L is a leaving group, such as F, Cl, Br, I, $NO_2$, 1,2,4-triazol-1-yl, O-$SiMe_3$, arylthio, alkylthio, alkylsulfonyl, arylsulfonyl, alkoxy, or arylsulfinyl. The general procedure is described in, e.g. Bull. Soc. Chim., 1969, 4344; Diss. Abstr. Int. B32 (12); 6915 (1972); and Ger. Offen. 1, 817, 146. A chloride of formula (23) wherein L is Cl is allowed to react with an appropriate amine at a wide variety of temperatures (20-180°C), preferably in the presence of an acid acceptor, such as triethylamine. The reaction may be carried out neat, or in a non-reactive

organic solvent. Compounds where $R^4$ is acyl are prepared from amines where $R^4$ is H, which were allowed to react with an acylating agent such as an acetyl chloride or acetic anhydride.

Synthesis of 4-chloro compounds of formula (3) is illustrated in, for example, J. Pharm. Soc. Jpn., 109, 464-473 (1989); Bull. Soc. Chim. 1975, 587-91; Bull. Soc. Chim. 1976, 761-764.

Synthesis of Compounds Wherein X is O

The compounds of formula (1) wherein X is O are made by condensing a compound of formula (23) with an alcohol or phenol of the formula (24):

HO-Y-Z (24) (24)

where

Y and Z are as previously defined.

The reaction is preferably carried out is the presence of a strong base, such as sodium hydride, in a non-reactive organic solvent, such as DMF, at a temperature in the range of 0 to 25°C.

Compounds wherein X is S are made by the same procedure using analogous mercaptans of formula HS-Y-Z.

Synthesis of Compounds Wherein X is $CR^5R^6$

The compounds of formula (1) wherein X is $CH_2$ can be made using the process described in the J. Heterocyclic Chemistry, Vol. 14, 1081-1083 (1977).

This procedure involves hydrolysis and decarboxylation of 5-substituted-5-(4-thienopyrimidyl)-barbituric acids of the formula

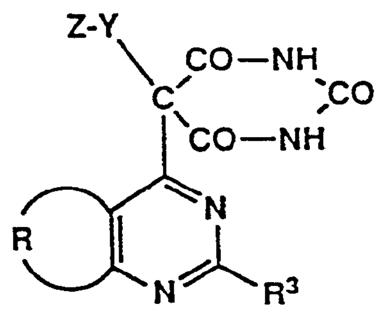

wherein the variable substituents are as defined for formula (1). The 5-substituted-5-(4-thienopyrimidyl)-barbituric acid is dissolved in a solution of sodium hydroxide and water and refluxed. The solution is then made slightly acidic and again refluxed.

Compounds of formula (1) wherein X is $CR^5R^6$ may also be prepared by reacting a compound of formula (23) where L is H with a Grignard reagent of the formula Z-Y-$CR^5R^6$-MgX′ or a lithio reagent of the formula Z-Y-$CR^5R^6$-Li, where X′ is halo, to provide a 3,4-dihydro-thienopyrimidine which is then oxidized to provide a compound of the invention. Typical reaction conditions are those described in Armarego and Smith, J. Chem. Soc., page 5360 (1965).

The acid addition salts and N-oxides of compounds of formula (1) are obtained in the usual way.

Accordingly, the invention also provides a process for preparing a compound of formula (1) which comprises

(a) condensing a compound of formula (23)

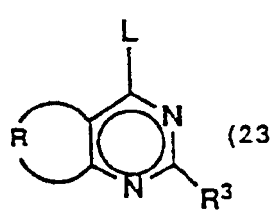

(23)

wherein R is as previously defined, and L is a leaving group, with an alcohol of the formula (8):

HO-Y-Z (8)

wherein Y and Z are as previously defined to produce a compound of formula (1) wherein X is O; or

(b) condensing a compound of formula (23) as defined above with an amine of the formula (9)

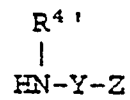

(9)

where $R^{4'}$ is H or ($C_1$-$C_4$) alkyl, and Y and Z are as previously defined, to provide a compound of formula (1) where X is $NR^{4'}$; or

(c) acylating a compound of formula (1) wherein X is NH to provide a compound of formula (1) wherein X is $NR^4$ and $R^4$ is acyl; or

(d) reacting a compound of formula (23) wherein L is H with a Grignard reagent of formula Z-Y-$CR^5R^6$-MgX', where X' is halo and Z and Y are as defined above, or with a lithio reagent of formula Z-Y-$CR^5R^6$-Li, to provide a 3,4-dihydro thienopyrimidine, which is then oxidized to produce a compound of formula (1) wherein X is $CR^5R^6$; or

(e) hydrolyzing and decarboxylating a 5-substituted-5-(4-(thienopyrimidinyl)barbituric acid of the formula

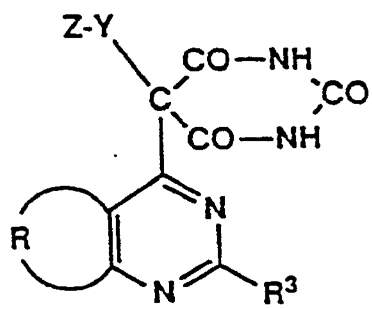

wherein R, $R^3$, $R^5$, $R^6$, Y, and Z are as previously defined to provide a compound of formula (1) wherein X is $CH_2$; or

(f) heating a compound of the formula (7)

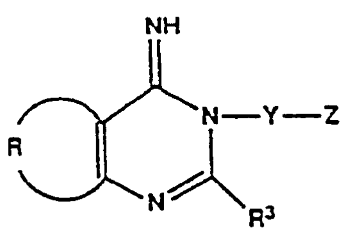

(7)

optionally in the presence of base to provide a compound of the formula (1d)

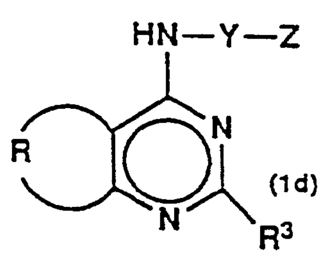

(1d)

Examples 1-72

The following compounds of formula (1) were actually prepared by the above described general procedures. The melting point is given for each compound. In addition, although the data has not been included, each compound was characterized by one or more of NMR, IR, mass spectra, and combustion analysis. Specific illustrative preparations for the compounds of representative compounds follow the tabular listing.

| EXAMPLE NUMBER | COMPOUND | M.P. |
|---|---|---|
| 1 | 4-[2-[4-(t-butyl)phenyl]ethoxy]-5,6,7,8-tetrahydrobenzothieno[2,3-d]-pyrimidine | 65-70°C |
| 2 | 4-[2-[4-(t-butyl)phenyl]ethoxy]-thieno[3,2-d]pyrimidine | 90-92°C |
| 3 | 4-(4-fluorophenoxy)thieno[3,2-d]-pyrimidine | 121-124°C |
| 4 | N-[2-[4-(1,1,2,2-tetrafluoro-ethoxy)phenyl]ethyl]thieno[2,3-d]-pyrimidin-4-amine | 153-155°C |
| 5 | 4-[2-(4-pentafluoroethoxyphenyl)-ethoxy]thieno[3,4-d]pyrimidine | 72-74°C |
| 6 | 4-[2-(1-adamantyl)ethoxy]thieno-[2,3-d]pyrimidine | 70-72°C |
| 7 | N-[3-[4-(cyclohexylmethoxy)phenyl]-1-ethylpropyl]thieno[3,2-d]pyrimidin-4-amine | 103-105°C |
| 8 | 4-[2-[4-(t-butyl)phenyl]ethoxy]-thieno[2,3-d]pyrimidine | 118-120°C |
| 9 | 4-(4-fluorophenoxy)thieno[2,3-d]-pyrimidine | 98-99°C |
| 10 | N-[2-[4-(i-propyl)phenyl]ethyl]-thieno[2,3-d]pyrimidin-4-amine | 150-151°C |
| 11 | N-[2-(4-methoxyphenyl)ethyl]thieno-[2,3-d]pyrimidin-4-amine | 167-168°C |
| 12 | N-[2-(4-chlorophenyl)ethyl]thieno-[2,3-d]pyrimidin-4-amine | 189-190°C |
| 13 | N-[2-[4-(1,1,2,2-tetrafluoro-ethoxy)phenyl]ethyl]thieno[3,2-d]-pyrimidin-4-amine | 145-147°C |

| EXAMPLE NUMBER | COMPOUND | M.P. |
|---|---|---|
| 14 | N-[2-(3-fluoro-4-methoxyphenyl)-ethyl]thieno[3,2-d]pyrimidin-4-amine | 152-153°C |
| 15 | N-[2-[4-(t-butyl)phenyl]ethyl]-thieno[3,4-d]pyrimidin-4-amine | 155-160°C |
| 16 | 4-[2-(4-methoxyphenyl)ethoxy]-thieno[3,4-d]pyrimidine | oil |
| 17 | N-[2-(4-chlorophenyl)ethyl]-thieno[3,2-d]pyrimidin-4-amine | 180-182°C |
| 18 | 4-[(4-fluorophenyl)thio]thieno-[3,2-d]pyrimidine | 132-134°C |
| 19 | 4-[2-(4-chlorophenyl)ethoxy]thieno-[3,2-d]pyrimidine | 112-144°C |
| 20 | 4-[2-[4-(trifluoromethyl)phenyl]-ethoxy]thieno[3,2-d]pyrimidine | 81-83°C |
| 21 | N-[2-[3-(trifluoromethyl)phenyl]-ethyl]thieno[3,2-d]pyrimidin-4-amine | 113-115°C |
| 22 | N-[1-ethyl-3-[4-(cyclohexylmethoxy)-phenyl]propyl]-5,6,7,8-tetrahydrobenzo-thieno[2,3-d]pyrimidin-4-amine | oil |
| 23 | N-[2-(2-naphthyl)ethyl]thieno-[2,3-d]pyrimidin-4-amine | 185-188°C |
| 24 | N-(4-fluorophenyl)thieno[3,2-d]-pyrimidin-4-amine | 196-198°C |
| 25 | 5-methyl-N-[2-(2-naphthyl)ethyl]-thieno[2,3-d]pyrimidin-4-amine | 106-107°C |
| 26 | N-methyl-N-[2-(4-methoxyphenyl)-ethyl]thieno[3,2-d]pyrimidin-4-amine | 106-107°C |
| 27 | N-[2-(2-methoxyphenyl)ethyl]thieno-[2,3-d]pyrimidin-4-amine | 131-132°C |

| EXAMPLE NUMBER | COMPOUND | M.P. |
|---|---|---|
| 28 | N-[2-(3-methoxyphenyl)ethyl]thieno-2,3-d]pyrimidin-4-amine | 145-146°C |
| 29 | N-[2-(3-fluoro-4-methoxyphenyl)-ethyl]thieno[2,3-d]pyrimidin-4-amine | 175-176°C |
| 30 | N-(1-methylbutyl)thieno[3,2-d]-pyrimidin-4-amine | 108-114°C |
| 31 | N-[2-[4-(t-butyl)phenyl]ethyl]-thieno[3,2-d]pyrimidin-4-amine | 132-135°C |
| 32 | N-[(2,2'-bithien-5-yl)methyl]-thieno[3,2-d]pyrimidin-4-amine | 149-151°C |
| 33 | 4-[2-(4-biphenylyl)ethoxy]-thieno[3,2-d]pyrimidine | 104-105°C |
| 34 | N-[2-(4-methoxyphenyl)ethyl]thieno-[3,2-d]pyrimidin-4-amine | 158-160°C |
| 35 | 4-[2-(4-ethoxyphenyl)ethoxy]thieno-[3,2-d]pyrimidine | 80-82°C |
| 36 | 4-[2-(4-methoxyphenyl)ethoxy]thieno-[3,2-d]pyrimidine | 61-63°C |
| 37 | N-[2-(4-ethoxyphenyl)ethyl]thieno-[3,2-d]pyrimidin-4-amine | 155-160°C |
| 38 | 4-[2-(2-naphthyl)ethoxy]thieno-[3,2-d]pyrimidine | 86-88°C |
| 39 | 4-[[4-(1,1,2,2-tetrafluoroethoxy)-phenyl]methoxy]thieno[3,2-d]pyrimidine | 55-57°C |
| 40 | cis-4-[2-(2,6-dimethyl-4-morpholinyl)-ethoxy]thieno[3,2-d]pyrimidine | oil |
| 41 | trans-4-[2-(2,6-dimethyl-4-morpholin-yl)ethoxy]thieno[3,2-d]pyrimidine | oil |

| EXAMPLE NUMBER | COMPOUND | M.P. |
|---|---|---|
| 42 | 4-(1-methylbutoxy)thieno[3,2-d]-pyrimidine | oil |
| 43 | 4-(2-chloro-4-fluorophenoxy)thieno-[3,2-d]pyrimidine | 153-155°C |
| 44 | N-methyl-N-(2-phenylethyl)thieno-[3,2-d]pyrimidin-4-amine | oil |
| 45 | 4-(1H-1,2,4-triazol-1-yl)thieno-[3,4-d]pyrimidine | 176-178°C |
| 46 | N-[2-[4-(t-butyl)phenyl]ethyl]-thieno[2,3-d]pyrimidin-4-amine | 130-131°C |
| 47 | N-[2-(4-methoxyphenyl)ethyl]thieno-[3,4-d]pyrimidin-4-amine | 185-186°C |
| 48 | 5-methyl-N-[2-(4-methoxyphenyl)-ethyl]thieno[2,3-d]pyrimidin-4-amine | 102-103°C |
| 49 | 4-[2-[4-[4-(trifluoromethyl)phenoxy]-phenyl]ethoxy]thieno[3,4-d]pyrimidine | oil |
| 50 | 4-[2-[4-[3-(trifluoromethyl)phenoxy]-phenyl]ethoxy]thieno[2,3-d]pyrimidine | 50-60°C |
| 51 | 4-[2-[4-(1,1,2,2-tetrafluoroethoxy)-phenyl]ethoxy]thieno[2,3-d]pyrimidine | 41-43°C |
| 52 | N-[2-[4-(2,2,2-trifluoroethoxy)-phenyl]ethyl]thieno[2,3-d]pyrimidin-4-amine | 149-150°C |
| 53 | 5-methyl-N-[2-[4-(1,1,2,2-tetra-fluoroethoxy)phenyl]ethyl]thieno-[2,3-d]pyrimidin-4-amine | 105-107°C |
| 54 | 5-methyl-4-[2-[3,4-(difluoromethyl-enedioxy)phenyl]ethoxy]thieno[2,3-d]-pyrimidine | 108-109°C |

| EXAMPLE NUMBER | COMPOUND | M.P. |
|---|---|---|
| 55 | N-[2-(1-cyclohexenyl)ethyl]-5-methylthieno[2,3-d]pyrimidin-4-amine | 70-71°C |
| 56 | N-[2-[3,4-(difluoromethylene-dioxy)phenyl]ethyl]thieno[2,3-d]pyrimidin-4-amine | 143-147°C |
| 57 | N-[2-[4-(2,2,2-trifluoroethoxy)-phenyl]ethyl]-5-methyl-thieno[2,3-d]pyrimidin-4-amine | 136-137°C |
| 58 | 4-[2-[4-(2,2,2-trifluoroethoxy)-phenyl]ethoxy]-5-methyl-thieno[2,3-d]pyrimidine | 71-74°C |
| 59 | 4-[3-[4-(methoxy)phenyl]propyl]-thieno[2,3-d]pyrimidine | 76-78°C |
| 60 | 4-[2-[4-(2,2,2-trifluoroethoxy)-phenyl]ethoxy]thieno[2,3-d]-pyrimidine | 74-76°C |
| 61 | 4-[3-[4-(t-butyl)phenyl]propyl]-5-methylthieno[2,3-d]pyrimidine | 91-93°C |
| 62 | N-(2-hydroxy-2-phenylethyl)-thieno[2,3-d]pyrimidin-4-amine | 181-183°C |
| 63 | 5-methyl-N-(2-phenylcyclopropyl)-thieno[2,3-d]pyrimidin-4-amine | 104-106°C |
| 64 | 2-methyl-N-[2-[4-(2,2,2-tri-fluoroethoxy)phenyl]ethyl]-5,6,7,8-tetrahydrobenzo-thieno[2,3-d]pyrimidin-4-amine | 107-108°C |
| 65 | 5-methyl-N-[2-[3-fluoro-4-(2,2,2-trifluoroethoxy)phenyl]ethyl]-thieno[2,3-d]pyrimidin-4-amine | 117-120°C |
| 66 | N-[2-[3-fluoro-4-(2,2,2-trifluoroethoxy)phenyl]ethyl]-thieno[2,3-d]pyrimidin-4-amine | 167-169°C |
| 67 | 5-methyl-N-methyl-N-[2-[4-(1,1,2,2-tetrafluoroethoxy)-phenyl]ethyl]thieno[2,3-d]-pyrimidin-4-amine | NA |

| | | |
|---|---|---|
| 68 | 5-methyl-N-[2-[4-(trimethyl-silyl)phenyl]ethyl]thieno[2,3-d]-pyrimidin-4-amine | 97-98°C |
| 69 | N-[2-[4-(trimethylsilyl)phenyl]-ethyl]thieno[2,3-d]-pyrimidin-4-amine | 133-135°C |
| 70 | N-[2-[4-(2,2,2-trifluoroethoxy)-phenyl]ethyl]-thieno[3,2-d]pyrimidin-4-amine | 150-151°C |
| 71 | N-[2-[4-(2,2,2-trifluoroethoxy)-phenyl]ethyl]-thieno[3,4-d]pyrimidin-4-amine | 165-166°C |
| 72 | N-[2-[4-(2,2,2-trifluoroethoxy)-phenyl]ethyl]-5-methyl-thieno[2,3-d]pyrimidine | 53-55°C |

The procedures described in the following detailed examples are representative of the procedures used to prepare the compounds of the other examples.

Preparation A

2-Amino-3-cyanothiophene

To 4.8 g (.032 m) of 2,5-dihydroxy-1,4-dithiane was added 2.8 g (.042 m) of malononitrile and 50 mL of ethanol. This mixture was treated with 4 g (.0495 m) of pyridine and heated at 50°C in a water bath with occasional stirring for six hours. The mixture was filtered to remove unreacted dithiane, and the filtrate was evaporated. The residue was dissolved in $CH_2Cl_2$ and water. The product was extracted into three portions of $CH_2Cl_2$, which were combined, washed three times with water, dried and evaporated down to give the product. Yield 5.2 g. M.P. 90-95°C.

Preparation B

4-Chlorothieno[3,2-d]pyrimidine

a) To 450 mL (4.77 m) of acetic anhydride cooled to 15°C was added in 25 mL portions 450 mL (11.9 m) of 97% formic acid over $\frac{1}{2}$ hour. During the addition the mixture was subjected to mild cold water cooling. When the addition was complete, the mixture was removed from the ice bath and stirred at room temperature for $\frac{1}{2}$ hour. To this mixture was then added 157 g (1 m) of methyl 3-aminothiophene-2-carboxylate in portions over 15 minutes. After allowing the mixture to react at room temperature for four hours, the mixture was evaporated down to give a precipitate, which was washed with ether to give methyl 3-formamidothiophene-2-carboxylate. Yield 148.1 g. M.P. 92-94°C.

b) To 86.5 g (.468 m) of methyl 3-formamidothiophene-2-carboxylate was added 86.5 g of ammonium formate and 380 mL of formamide. The mixture was stirred for five hours at 140-165°C, then cooled to 10°C, and filtered to collect thieno[3,2-d]pyrimidin-4-one. Yield 41.4 g MP 90-91°C.

c) To 9.4 g (.062 m) of thieno[3,2-d]pyrimidin4-one was added 80 mL of $POCl_3$. The mixture was refluxed for three hours with stirring, and then allowed to react overnight at room temperature. The mixture was evaporated down, and the residue treated cautiously with ice to hydrolyze excess $POCl_3$. Then concentrated $NH_4OH$ was added until the pH of the mixture was 6. The precipitate was collected by filtration and washed with water, providing 9.9 g of the desired 4-chlorothieno[3,2-d]-pyrimidine. M.P. 117-119°C.

Preparation C

2-Amino-3-cyano-4-methylthiophene

To 8.3 g (.123 m) malononitrile and 12.5 g (069 m) of 2,5-dihydroxy-2,5-dimethyl-1,4-dithiane in 12.5 mL of ethanol was added 125 mL of piperidine. The mixture was stirred overnight at room temperature, then concentrated by evaporation. The residue was taken up in methylene chloride, which was then evaporated to give a precipitate. This was collected and washed with a small amount of methylene chloride to give the title product. Yield 10 g M.P. 119-120°C.

Preparation D

4-Chloro[2,3-d]thienopyrimidine

a) To 175 g (1.15 m) of 2,5-dihydroxy-1,4-dithiane and 237 g (2.1 m) of ethyl cyanoacetate was added 2000 mL of ethanol followed by 207 mL (2.1 m) of piperidine at slow stream rate. After sitting at room temperature overnight, the mixture was filtered to remove insoluble polymeric impurities. The ethanol filtrate was concentrated by evaporation and the residue dissolved in diethyl ether. The diethyl ether solution was decanted to remove remaining impurities, then concentrated by evaporation to 282 g of ethyl 2-aminothiophene-3-carboxylate.

b) To 470 mL of acetic anhydride at 10°C was added 470 mL of 97% formic acid in 25 mL portions. The mixture was maintained at a temperature of 25-30°C by mild cooling. To this mixture was added 282 g of ethyl 2-aminothiophene-3-carboxylate in portions. After stirring the mixture at room temperature for four hours, it was concentrated by evaporation. The residue was treated with diethyl ether and filtered to give 100.8 g of ethyl 2-formamidothiophene-3-carboxylate M.P. 57-60°C.

c) To 100 g of ethyl 2-formamidothiophene-3-carboxylate was added 100 g of ammonium formate and 400 mL of formamide. This mixture was heated with stirring at 140-145°C for five hours. The mixture was then cooled. The resulting precipitate was collected by filtration, washed with water and then $CH_2Cl_2$, and dried, giving 46.8 g of thieno[2,3-d]pyrimidin-4-one. M.P. 259-261°C.

d) To 42 g (.28 m) of thieno[2,3-d]pyrimidin-4-one was added 210 mL (2.25 m) of phosphorus oxychloride. This was heated to reflux for three hours with stirring, then cooled. Excess phosphorus oxychloride was removed by evaporation. Then ice was cautiously added to the mixture to hydrolyze remaining phosphorus oxychloride. Then sufficient concentrated ammonium hydroxide was cautiously added to bring the pH to 6. The resulting precipitate was collected, washed with water, and treated with $CH_2Cl_2$. Insoluble material (20.3 g) was isolated and identified as unreacted starting material. The $CH_2Cl_2$ solution was evaporated to provide 22 g of the desired product 4-chlorothieno[2,3-d]pyrimidine. M.P. 103-104°C.

Preparation E

4-Chloro-5-Methylthieno[2,3-d]pyrimidine

a) To 17 g of 2,5-dihydroxy-2,5-dimethyl-1,4-dithiane and 17 g of methyl cyanoacetate in 170 mL of ethanol was added 14.6 g of piperidine in portions. The mixture was heated to 50°C with stirring for one hour. The mixture was then concentrated, and treated with diethyl ether. The solids were collected, and washed with cold diethyl ether to give 18.1 g of methyl 2-amino-4-methylthiophene-3-carboxylate.

b) To 40 mL of acetic anhydride was slowly added 40 mL of 97% formic acid while holding the temperature at 25-30°C with mild ice cooling. Then 24 g of methyl 2-amino-4-methylthiophene-3-carboxylate was added, and the mixture was stirred for hour hours at room temperature. The mixture was then concentrated by evaporation, and the residue was treated with diethyl ether and filtered to give 26.2 g of methyl 2-formamido-4-methylthiophene-3-carboxylate. M.P. 78-81°C.

c) To 26 g of methyl 2-formamido-4-methylthiophene-3-carboxylate was added 26 g of ammonium formate and 100 mL of formamide. This mixture was heated with stirring for five hours at 140-145°C, then cooled to 5°C. The product, 5-methylthieno[2,3-d]pyrimidin-4-one, was collected by filtration and washed with $CH_2Cl_2$. Yield 6.9 g M.P. 246-248°C.

d) To 65 g of 5-methylthieno[2,3-d]pyrimidin-4-one was added 50 ML of $POCl_3$, and the mixture was refluxed for three hours, then cooled and concentrated to give a solid. This was cautiously treated with ice and water to hydrolyze the residual $POCl_3$. The pH of the mixture was adjusted to 6 by addition of concentrated ammonium hydroxide. The resulting precipitate was filtered and washed with water to give 7 g of 4-chloro-5-methylthieno[2,3-d]pyrimidine M.P. 135-136°C.

Example 5

4-[2-[4-(Pentafluoroethoxy)phenyl]ethoxy]-thieno[3,4-d]pyrimidine

To 3 mL of DMF was added .11 g of 60% NaH in mineral oil followed by .64 g of 2-[4-(pentafluoroethoxy)-phenyl]ethanol. After ½ hour .51 g of 4-(1H-1,2,4-triazol-1-yl)thieno[3,4-d]pyrimidine was added and the mixture was stirred at room temperature overnight. Then water and ethyl acetate were added to the mixture. The product was extracted into two fractions of ethyl acetate, which were combined and washed twice with water and twice with sodium chloride solution. The solution was then concentrated to provide .8 g of crude product. This was chromatographed on an $SiO_2$ column to give. 15 a of the title product. M.P. 70-72°C.

Example 11

N-[2-(4-methoxyphenyl)ethyl]thieno[2,3-d]-pyrimidin-4-amine

To 7.5 g (.0604 m) of 2-amino-3-cyanothiophene was added 115 mL of triethylorthoformate, and the mixture was heated for one hour as ethanol distilled off. The mixture was then concentrated by evaporation to give an oily residue. To this was added 7.6 g of sodium acetate and 9 g (.06 m) of 2-(4-methoxyphenyl)ethylamine. To this was then added 12 mL of acetic acid and the mixture was heated at 115-125°C for one hour as ethanol boiled off. The mixture was then cooled and treated with diethyl ether and water, and 10.4 g of crude product were isolated from this mixture by filtration. Additional product was extracted from the filtrate into three fractions of diethyl ether. These fractions were combined, washed three times with water to give an additional 1.5 g of crude product, which was combined with the initial 10.4 g. The crude product was treated with hot $CHCl_3$ and filtered. The filtrate was concentrated by evaporation to begin crystallization and cooled using dry ice/acetone. The precipitate was collected by filtration and washed with cold $CHCl_3$ to give the title product. Yield 5 g. M.P. 169-170°C. A second crop of crystals was collected. Yield 1.4 g M.P. 168-170°C.

Example 13

N-[2-[4-(2,2,3,3-tetrafluoroethoxy)phenyl]-ethyl]thieno[3,2-d]pyrimidin-4-amine

To 8.5 g of 4-chlorothieno[3,2-d]pyrimidine was added 1.2 g of 2-[4-(2,2,3,3-tetrafluoroethoxy)phenyl]ethylamine. These were heated to 150°C for five to ten minutes. The mixture was then cooled and extracted into ethyl acetate and water. This mixture was washed twice with water, twice with sodium chloride solution, and filtered. The filtrate was concentrated by evaporation to give 1.5 g of crude product, which was purified by chromatography on a silica gel column to give the desired product. Yield 0.5 g M.P. 145-147°C.

Example 35

4-[2-(4-Ethoxyphenyl)ethoxy]thieno[2,3-d]pyrimidine

To 10 mL of DMF was added .44 g of 60% NaH. After ½ hour, 1.7 g of 2-(4-ethoxyphenyl)ethanol was added, and the mixture was stirred for ½ hour. Then 1.7 g of 4-chlorothieno[3,2-d]pyrimidine was added and the mixture was stirred at room temperature overnight before adding water. The precipitate was collected, providing 2.3 g of crude product. M.P. 80-85°C. The crude product was recrystallized from acetone to give 1.3 g of purified product. M.P. 80-82°C.

Example 45

4-(1,2,4-Triazol-1-yl)thieno[3,4-d]pyrimidine

a) To 64 mL of acetic anhydride cooled to 10°C was added 38 mL of 97°% formic acid. The mixture was then brought to room temperature. To this mixture was added 10 g of methyl 3-aminothiophene-3-carboxylate hydrochloride. After allowing the reaction to proceed for ½ hour, the mixture was concentrated by evaporation. The residue was treated with 500 mL of boiling heptane, which was then filtered to remove insoluble tar. The filtrate was concentrated by evaporation to provide 8.7 g of methyl 3-formamidothiophene-4-carboxylate. M.P. 92-94°C.

b) To 8.0 g of methyl 3-formamidothiophene-4-carboxylate was added 8 g of ammonium formate and 35 mL of formamide. This was heated to 140-145°C for five hours, then cooled and concentrated by evaporation. The product was collected by filtration, providing 2.8 g of thieno[3,4-d]pyrimidin-4-one. M.P. 275-277°C.

c) To 1 g (.0066 m) of thieno[3,4-d]pyrimidin-4-one and 136 g (.0197 m) of 1H-1,2,4-triazole in 32.9 mL of pyridine was added 16 mL of 4-chlorophenyldichlorophosphate under nitrogen. The mixture was stirreed at room temperature under nitrogen overnight, then concentrated to dryness. The residue was treated with 500 mL of $CH_2Cl_2$. This solution was washed with water and evaporated to give a crude product that was found to include some residual starting material. About 1 g of the crude product was treated with 250 mL of hot $CH_2Cl_2$, and filtered to remove the insoluble starting material. The filtrate was concentrated by evaporation to provide the desired product, 4-(1H-1,2,4-triazol-1-yl)thieno[3,4-d]-pyrimidine. M.P. 172-174°C.

Example 48

5-Methyl-N-[2-(4-methoxyphenyl)ethyl]-thieno[2,3-d]pyrimidin-4-amine

To 1.1 g of 2-amino-3-cyano-4-methylthiophene was added 15 mL of triethylorthoformate. This mixture was heated to 120°C for one hour. Then the mixture was evaporated down and 1 g of sodium acetate and 1.2 g of N-[2-(4-methoxyphenyl)ethyl]amine were added, followed by 1.6 mL of acetic acid. The mixture was then heated again for one hour at 90-115°C. Then the mixture was cooled and treated with diethyl ether and water. The product was extracted into three fractions of diethyl ether which were combined, and evaporated. The precipitate was washed with a small amount of diethyl ether to give the title product. Yield 0.2 g. M.P. 102-103°C.

Examples 73-79

The following compounds of formula (7) were prepared by the procedure described in the detailed example below.

| EXAMPLE NUMBER | COMPOUND | M.P. |
|---|---|---|
| 73 | 3-[2-(4-methoxyphenyl)ethyl]-thieno[2,3-d]pyrimidin-4(3H)-imine | 135-137°C |
| 74 | 3-[2-(2-naphthyl)ethyl]thieno-[2,3-d]pyrimidin-4(3H)-imine | 171-173°C |
| 75 | 3-[2-[4-(t-butyl)phenyl]ethyl]-thieno[2,3-d]pyrimidin-4(3H)-imine | 150-151°C |
| 76 | 5-methyl-3-[2-(4-methoxyphenyl)-ethyl]thieno[2,3-d]pyrimidin-4(3H)-imine | 86-89°C |
| 77 | 3-[2-[4-(1,1,2,2-tetrafluorethoxy)-phenyl]ethyl]thieno[2,3-d]pyrimidin-4(3H)-imine | 115-119°C |
| 78 | 5-methyl-3-[2-[4-(1,1,2,2-tetra-fluorethoxy)phenyl]ethyl]thieno-[2,3-d]pyrimidin-4(3H)-imine | 91-93°C |
| 79 | 5-methyl-3-[2-[4-(2,2,2-trifluoro-ethoxy)phenyl]ethyl]thieno[2,3-d]pyrimidin-4(3H)-imine | 131-134°C |

Example 74

3-[2-(2-naphthyl)ethyl]thieno-[2,3-d]pyrimidin-4(3H)-imine

To 1 g (.008 mole) of 2-amino-3-cyanothiophene was added 15 mL of trimethyl orthoformate. The mixture was heated for one hour, then cooled and concentrated by evaporation. To the resulting oil was added 1.7 g (.008 mole) of 2-(2-naphthyl)ethylamine carbonate and 15 mL of benzene. When this did not produce a solution, 15 mL of methanol was added, which did provide a solution. The solution was stirred overnight at room temperature, then concentrated by evaporation to provide a crude product, which was recrystallized from $CH_2Cl_2$. Yield: 0.3 g. M.P. 171-173°C.

Fungicide Utility

The compounds of the present invention have been found to control fungi, particularly plant pathogens. When employed in the treatment of plant fungal diseases, the compounds are applied to the plants in a disease inhibiting and phytologically acceptable amount. The term "disease inhibiting and phytologically acceptable amount," as used herein, refers to an amount of a compound of the invention which kills or inhibits the plant disease for which control is desired, but is not significantly toxic to the plant. This amount will generally be from about 1 to 1000 ppm, with 10 to 500 ppm being preferred. The exact concentration of compound required varies with the fungal disease to be controlled, the type formulation employed, the method of application, the particular plant species, climate conditions and the like. A suitable application rate is typically in the range from .25 to 4 1b/A. The compounds of the invention may also be used to protect stored grain and other non-plant loci from fungal infestation.

Greenhouse Tests

The following experiments were performed in the laboratory to determine the fungicidal efficacy of the compounds of the invention.

Test 1

This screen was used to evaluate the efficacy of the present compounds against a variety of different organisms that cause plant diseases.

The test compounds were formulated for application by dissolving 50 mg of the compound into 125 ml of solvent. The solvent was prepared by mixing 50 ml of "Tween 20" (polyoxyethylene (20) sorbitan monolaurate emulsifier) with 475 ml of acetone and 475 ml of ethanol. The solvent/compound solution was diluted to 125 ml with deionized water. The resulting formulation contains 400 ppm test chemica. Lower concentrations were obtained by serial dilution with the solvent-surfactant mixture.

The formulated test compounds were applied by foliar spray. The following plant pathogens and their corresponding plants were employed.

| Pathogen | Designation in Following Table | Host |
|---|---|---|
| Erysiphe graminis tritici (powdery mildew) | POWD MDEW | wheat |
| Pyricularia oryzae (rice blast) | RICE BLAS | rice |
| Puccinia recondita tritici (leaf rust) | LEAF RUST | wheat |
| Botrytis cinerea (gray mold) | GRAY MOLD | grape berries |
| Pseudoperonospora cubensis (downy mildew) | DOWN MDEW | squash |

The formulated technical compounds were sprayed on all foliar surfaces of the host plants (or cut berry) to past run-off. Single pots of each host plant were placed on raised, revolving pedestals in a fume hood. Test solutions were sprayed on all foliar surfaces. All treatments were allowed to dry and the plants were inoculated with the appropriate pathogens within 2-4 hours.

The following table presents the activity of tyical compounds of the present invention when evaluated in this experiment. The effectiveness of test compounds in controlling disease was rated using the following scale.

| | | |
|---|---|---|
| 0 | = | not tested against specific organism |
| - | = | 0-19% control at 400 ppm |
| + | = | 20-89% control at 400 ppm |
| ++ | = | 90-100% control at 400 ppm |
| +++ | = | 90-100% control at 100 ppm |

## PLANT PATHOLOGY SCREEN

| EXAMPLE NUMBER | POWD MDEW | RICE BLAST | LEAF RUST | GRAY MOLD | DOWN MDEW |
|---|---|---|---|---|---|
| 1 | - | - | - | - | - |
| 2 | - | + | - | - | - |
| 3 | - | + | + | - | ++ |
| 4 | +++ | ++ | +++ | - | +++ |
| 5 | ++ | + | +++ | - | ++ |
| 6 | - | - | - | - | + |
| 7 | - | + | +++ | - | + |
| 8 | - | - | - | - | - |
| 9 | - | - | ++ | - | ++ |
| 10 | + | - | +++ | - | + |
| 11 | +++ | - | +++ | - | +++ |
| 12 | ++ | + | +++ | - | + |
| 13 | ++ | ++ | +++ | - | +++ |
| 14 | +++ | + | +++ | - | +++ |
| 15 | - | - | + | - | +++ |
| 16 | + | ++ | ++ | - | ++ |
| 17 | - | - | +++ | - | + |
| 18 | - | - | + | - | - |
| 19 | - | ++ | + | - | - |
| 20 | + | + | ++ | - | + |
| 21 | - | - | +++ | - | + |
| 22 | - | - | + | - | ++ |
| 23 | +++ | - | +++ | - | + |
| 24 | - | ++ | + | - | + |

PLANT PATHOLOGY SCREEN

| EXAMPLE NUMBER | POWD MDEW | RICE BLAST | LEAF RUST | GRAY MOLD | DOWN MDEW |
|---|---|---|---|---|---|
| 25 | +++ | + | +++ | 0 | +++ |
| 26 | - | - | + | - | ++ |
| 27 | - | - | ++ | - | +++ |
| 28 | - | - | ++ | - | +++ |
| 29 | - | - | ++ | - | + |
| 30 | ++ | + | +++ | - | +++ |
| 31 | ++ | ++ | +++ | - | +++ |
| 32 | - | + | ++ | - | + |
| 33 | - | + | + | - | + |
| 34 | ++ | - | +++ | - | +++ |
| 35 | - | +++ | +++ | - | +++ |
| 36 | + | + | ++ | - | +++ |
| 37 | ++ | + | +++ | - | ++ |
| 38 | ++ | - | +++ | - | + |
| 39 | - | - | - | - | + |
| 40 | + | - | + | - | - |
| 41 | - | - | + | - | + |
| 42 | - | - | + | - | +- |
| 43 | - | - | - | - | - |
| 44 | + | + | ++ | - | + |
| 45 | - | - | + | - | - |
| 46 | ++ | - | +++ | - | +++ |
| 47 | + | - | ++ | - | +++ |
| 48 | - | +++ | +++ | - | +++ |

PLANT PATHOLOGY SCREEN

| EXAMPLE NUMBER | POWD MDEW | RICE BLAST | LEAF RUST | GRAY MOLD | DOWN MDEW |
|---|---|---|---|---|---|
| 49 | - | - | - | - | +++ |
| 50 | +++ | +++ | +++ | - | +++ |
| 51 | +++ | +++ | +++ | - | +++ |
| 52 | +++ | +++ | +++ | - | +++ |
| 53 | +++ | ++ | +++ | - | +++ |
| 54 | + | - | + | - | + |
| 55 | ++ | ++ | ++ | - | + |
| 57 | + | - | +++ | - | ++ |
| 58 | +++ | +++ | +++ | - | +++ |
| 59 | + | + | + | - | ++ |
| 60 | +++ | +++ | +++ | - | ++ |
| 61 | + | + | + | 0 | + |
| 62 | - | - | + | 0 | + |
| 63 | ++ | - | - | 0 | + |
| 64 | - | - | - | 0 | + |
| 65 | + | ++ | +++ | 0 | 0 |
| 66 | ++ | +++ | +++ | 0 | 0 |
| 67 | +++ | ++ | +++ | 0 | 0 |
| 68 | +++ | + | +++ | 0 | + |
| 69 | +++ | ++ | +++ | 0 | 0 |
| 71 | + | + | ++ | | |
| 72 | ++ | ++ | ++ | 0 | 0 |

PLANT PATHOLOGY SCREEN - continued

| EXAMPLE NUMBER | POWD MDEW | RICE BLAST | LEAF RUST | GRAY MOLD | DOWN MDEW |
|---|---|---|---|---|---|
| 73 | - | + | ++ | - | +++ |
| 74 | - | + | + | - | +++ |
| 75 | - | - | + | - | +++ |
| 76 | + | + | ++ | - | +++ |
| 77 | ++ | +++ | ++ | - | +++ |
| 78 | + | ++ | ++ | - | +++ |
| 79 | + | + | + | - | ++ |

## Combinations

Fungal disease pathogens are known to develop resistance to fungicides. When strains resistant to a fungicide do develop, it becomes necessary to apply larger and larger amounts of the fungicide to obtain desired results. To retard the development of resistance to new fungicides, it is desirable to apply the new fungicides in combination with other fungicides. Use of a combination product also permits the product's spectrum of activity to be adjusted.

Accordingly, another aspect of the invention is a fungicidal combination comprising at least 1% by weight of a compound of formula (1) in combination with a second fungicide.

Contemplated classes of fungicides from which the second fungicide may be selected include:

1) N-substituted azoles, for example propiconazole, triademefon, flusilazol, diniconazole, ethyltrianol, myclobutanil, and prochloraz;

2) pyrimidines, such as fenarimol and nuarimol;

3) morpholines, such as fenpropimorph and tridemorph;

4) piperazines, such as triforine; and

5) pyridines, such as pyrifenox.

Fungicides in these five classes all function by inhibiting sterol biosynthesis. Additional classes of contemplated fungicides, which have other mechanisms of action include:

6) dithiocarbamates, such as maneb and mancozeb;

7) phthalimides, such as captafol;

8) isophthalonitrites, such as chlorothalonil;

9) dicarboximides, such as iprodione;

10) benzimidazoles, such as benomyl and carbendazim;

11) 2-aminopyrimidines, such as ethirimol;

12) carboxamides, such as carboxin; and

13) dinitrophenols, such as dinocap.

The fungicide combinations of the invention contain at least 1%, ordinarily 20 to 80%, and more typically 50 to 75% by weight of a compound of formula (1).

## Insecticide and Miticide Utility

The compounds of the invention are also useful for the control of insects and mites. Therefore, the present invention also is directed to a method for inhibiting an insect or mite which comprises applying to a locus of the insect or mite an insect- or mite-inhibiting amount of a compound of formula (1).

The compounds of the invention shown activity against a number of insects and mites. More specifically, the compounds show activity against melon aphid, which is a member of the insect order Homoptera. Other members of the Homoptera include leafhoppers, planthoppers, pear pyslla, apple sucker, scale insects, whiteflies, spittle bugs as well as numerous other host specific aphid species. Activity has also been observed against greenhouse thrips, which are members of the order Thysanoptera. The compounds also show activity against

Southern armyworm, which is a member of the insect order Lepidoptera. Other typical members of this order are codling moth, cutworm, clothes moth, Indianmeal moth, leaf rollers, corn earworm, European corn borer, cabbage worm, cabbage looper, cotton bollworm, bagworm, eastern tent caterpillar, sod webworm, and fall armyworm.

Representative mite species with which it is contemplated that the present invention can be practiced include those listed below.

| FAMILY | SCIENTIFIC NAME | COMMON NAME |
|---|---|---|
| ACARIDAE | | |
| | *Aleurobius farinae* | |
| | *Rhizoglyphus echinopus* | Bulb mite |
| | *Rhizoglyphus elongatus* | |
| | *Rhizoglyphus rhizophagus* | |
| | *Rhizoglyphus sagittatae* | |
| | *Rhizoglyphus tarsalis* | |
| ERIOPHYIDAE | | |
| | *Abacarus farinae* | Grain rust mite |
| | *Aceria brachytarsus* | |
| | *Acalitus essigi* | Redberry mite |
| | *Aceria ficus* | |
| | *Aceria fraaxinivorus* | |
| | *Aceria granati* | |
| | *Aceria parapopuli* | |
| | *Eriophyes sheldoni* | Citrus bud mite |
| | *Aceria tulipae* | |
| | *Aculus carnutus* | Peach silver mite |
| | *Aculus schlechtendali* | Apple rust mite |
| | *Colomerus vitis* | Grape erineum mite |
| | *Eriophyes convolvens* | |
| | *Eriophyes insidiosus* | |
| | *Eriophyes malifoliae* | |
| | *Eriophyes padi* | |
| | *Eriophyes pruni* | |
| | *Epitrimerus pyri* | Pear leaf blister mite |
| | *Eriophyes ramosus* | |

| FAMILY | SCIENTIFIC NAME | COMMON NAME |
|---|---|---|
| | *Eriophyes sheldoni* | Citrus bud mite |
| | *Eriophyes ribis* | |
| | *Phyllocoptes gracilis* | Dryberry mite |
| | *Phyllocoptruta oleivora* | Citrus rust mite |
| | *Phytoptus ribis* | |
| | *Trisetacus pini* | |
| | *Vasates amygdalina* | |
| | *Vasates eurynotus* | |
| | *Vasates quadripedes* | Maple bladdergall mite |
| | *Vasates schlechtendali* | |
| EUPODIDAE | | |
| | *Penthaleus major* | Winter grain mite |
| | *Linopodes spp.* | |
| NALEPELLIDAE | | |
| | *Phylocoptella avellanae* | Filbert bud mite |
| PENTHALEIDAE | | |
| | *Halotydeus destrustor* | |
| PYEMOTIDAE | | |
| | *Pyemotes tritici* | Straw itch mite |
| | *Siteroptes cerealium* | |
| TARSONEMIDAE | | |
| | *Polyphagotarsonemus latus* | Broad mite |
| | *Steneotarsonemus pallidus* | Cyclamen mite |

| FAMILY | SCIENTIFIC NAME | COMMON NAME |
|---|---|---|
| TENUIPALPIDAE | | |
| | Brevipalpus californicus | |
| | Brevipalpus obovatus | Privet mite |
| | Brevipalpus lewisi | Citrus flat mite |
| | Dolichotetranychus floridanus | Pineapple flase spider mite |
| | Tenuipalpes granati | |
| | Tenuipalpes pacificus | |
| TETRANYCHIDAE | | |
| | Bryobia arborea | |
| | Bryobia practiosa | Clover mite |
| | Bryobia rubrioculus | Brown mite |
| | Eotetranychus coryli | |
| | Eotetranychus hicoriae | Pecan deaf scorch mite |
| | Eotetranychus lewisi | |
| | Eotetranychus sexmaculatus | Sixspotted spider mite |
| | Eotetranychus willametti | |
| | Eotetranychus banksi | Texas citrus mite |
| | Oligonychus ilicis | Southern red mite |
| | Oligonychus pratensis | Banks grass mite |
| | Oligonychus ununguis | Spruce spider mite |
| | Panonychus citri | Citrus red mite |
| | Panonychus ulmi | European red mite |
| | Paratetranychus modestus | |
| | Paratetranychus pratensis | |
| | Paratetranychus viridis | |
| | Petrobia latens | Brown wheat mite |

| FAMILY | SCIENTIFIC NAME | COMMON NAME |
|---|---|---|
| | Schizotetranychus celarius | Bamboo spider mite |
| | Schizotetranychus pratensis | |
| | Tetranychus canadensis | Fourspotted spider mite |
| | Tetranychus cinnabarinus | Carmine spider mite |
| | Tetranychus mcdanieli | McDaniel spider mite |
| | Tetranychus pacificus | Pacific spider mite |
| | Tetranychus schoenei | Schoene spider mite |
| | Tetranychus urticae | Twospotted spider mite |
| | Tetranychus turkestani | Strawberry spider mite |
| | Tetranychus desertorum | Desert spider mite |

The compounds are useful for reducing populations of insects and mites, and are used in a method of inhibiting an insect or mite population which comprises applying to a locus of the insect or arachnid an effective insect- or mite-inactivating amount of a compound of formula (1). The "locus" of insects or mites is a term used herein to refer to the environment in which the insects or mites live or where their eggs are present, including the air surrounding them, the food they eat, or objects which they contact. For example, plant-ingesting insects or mites can be controlled by applying the active compound to plant parts, which the insects or mites eat, particularly the foliage. It is contemplated that the compounds might also be useful to protect textiles, paper, stored grain, or seeds by applying an active compound to such substance. The term "inhibiting an insect or mite" refers to a decrease in the numbers of living insects or mites; or a decrease in the number of viable insect or mite eggs. The extent of reduction accomplished by a compound depends, of course, upon the application rate of the compound, the particular compound used, and the target insect or mite species. At least an insect-inactiviting or mite-inactivating amount should be used. The terms "insect-inactivating amount" and "mite-inactivating amount" are used to describe the amount, which is sufficient to cause a measurable reduction in the treated insect or mite population. Generally an amount in the range from about 1 to about 1000 ppm active compound is used.

In a preferred embodiment, the present invention is directed to a method for inhibiting a mite which comprises applying to a plant an effective mite-inactivating amount of a compound of formula (1) in accordance with the present invention.

## MITE/INSECT SCREEN

The compounds of Examples 1-60 were tested for miticidal and insecticidal activity in the following mite/insect screen.

Each test compound was formulated by dissolving the compound in acetone/alcohol (50:50) mixture containing 23 g of "TOXIMUL R" (sulfonate/nonionic emulsified blend) and 13 g of "TOXIMUL S" (sulfonate/nonionic emulsifier blend) per liter. These mixtures were then diluted with water to give the indicated concentrations.

Twospotted spider mites (Tetranychus urticae Koch) and melon aphids (Aphis gossypii Glover) were introduced on squash cotyledons and allowed to establish on both leaf surfaces. Other plants in the same treatment pot were left uninfested. The leaves were then sprayed with 5 ml of test solution using a DeVilbiss atomizing sprayer at 10 psi. Both surfaces of the leaves were covered until runnoff, and then allowed to dry for one hour. Two uninfested leaves were then excised and placed into a Petri dish containing larval southern armyworm (Spodopetra eridania Cramer).

Activity on Southern corn rootworm (Diabrotica undecimpuctata howardi Barber) was evaluated by adding two ml of tap water, a presoaked corn seed, and 15 g of dry sandy soil to a one ounce plastic container. The soil was treated with 1 mL of test solution containing a predetermined concentration of test compound. After six to 12 hours of drying, five 2-3 instar corn rootworm larvae were added to the individual cups, which were then capped and held at 23°C.

After standard exposure periods, percent mortality and phototoxicity were evaluated. Results for the compounds found to be active are reported in the following table. The remaining compounds showed no activity. The following abbreviations are used in the table:

CRW refers to corn rootworm
SAW refers to Southern armyworm
SM refers to twospotted spider mites
MA refers to melon aphids.

| COMPOUND | CRW RATE PPM | CRW % | SAW SM & MA RATE PPM | SAW % | SM % | MA % |
|---|---|---|---|---|---|---|
| 1 | 12.00 | 0 | 200 | 0 | 100 | 100 |
| | 24.00 | 0 | 400 | 0 | 0 | 0 |
| 2 | 12.00 | 0 | 200 | 0 | 80 | 100 |
| | 24.00 | 0 | 400 | 0 | 80 | 80 |
| 3 | 12.00 | 0 | 200 | 0 | 0 | 0 |
| | 24.00 | 0 | 400 | 0 | 0 | 0 |
| 4 | 12.00 | 0 | 200 | 0 | 20 | 0 |
| | 24.00 | 0 | 400 | 0 | 100 | 100 |
| 5 | 12.00 | 0 | 200 | 0 | 100 | 100 |
| | 24.00 | 0 | 400 | 0 | 60 | 80 |
| 6 | 12.00 | 0 | 200 | 0 | 0 | 0 |
| | 24.00 | 0 | 400 | 0 | 0 | 0 |
| 7 | 12.00 | 0 | 200 | 0 | 0 | 0 |
| | 24.00 | 0 | 400 | 0 | 0 | 0 |
| 8 | 12.00 | 0 | 200 | 0 | 90 | 0 |
| | 24.00 | 0 | 400 | 0 | 80 | 40 |
| 9 | 12.00 | 0 | 200 | 0 | 50 | 50 |
| | 24.00 | 0 | 400 | 0 | 0 | 0 |
| 10 | 12.00 | 0 | 200 | 0 | 90 | 90 |
| | 24.00 | 40 | 400 | 0 | 0 | 0 |
| 11 | 12.00 | 0 | 200 | 0 | 0 | 70 |
| | 24.00 | 0 | 400 | 0 | 0 | 0 |
| 12 | 12.00 | 0 | 200 | 0 | 0 | 60 |
| | 24.00 | 100 | 400 | 0 | 20 | 40 |
| 13 | 12.00 | 0 | 200 | 60 | 100 | 100 |
| | 24.00 | 100 | 400 | 0 | 100 | 90 |

| COMPOUND | CRW RATE PPM | CRW % | SAW SM & MA RATE PPM | SAW % | SM % | MA % |
|---|---|---|---|---|---|---|
| 14 | 12.00 | 0 | 200 | 0 | 0 | 0 |
| | 24.00 | 0 | 400 | 0 | 0 | 0 |
| 15 | 12.00 | 0 | 200 | 0 | 0 | 0 |
| | 24.00 | 0 | 400 | 0 | 0 | 0 |
| 16 | 12.00 | 0 | 200 | 0 | 50 | 80 |
| | 24.00 | 0 | 400 | 0 | 0 | 80 |
| 17 | 12.00 | 0 | 200 | 0 | 0 | 0 |
| | 24.00 | 0 | 400 | 0 | 0 | 0 |
| 18 | 24.00 | 60 | 400 | 0 | 0 | 0 |
| 19 | 12.00 | 0 | 200 | 0 | 0 | 0 |
| | 24.00 | 0 | 400 | 0 | 0 | 0 |
| 20 | 12.00 | 0 | 200 | 0 | 0 | 0 |
| | 24.00 | 0 | 400 | 0 | 0 | 100 |
| 21 | 12.00 | 0 | 200 | 0 | 0 | 90 |
| | 24.00 | 0 | 400 | 0 | 0 | 100 |
| 22 | 12.00 | 0 | 200 | 0 | 0 | 0 |
| | 24.00 | 0 | 400 | 0 | 0 | 0 |
| 23 | 12.00 | 0 | 200 | 0 | 90 | 80 |
| | 24.00 | 0 | 400 | 0 | 50 | 50 |
| 24 | 12.00 | 0 | 200 | 0 | 0 | 0 |
| | 24.00 | 0 | 400 | 0 | 0 | 0 |
| 25 | 12.00 | 0 | 200 | 20 | 100 | 100 |
| | 24.00 | 0 | 400 | 0 | 0 | 100 |
| 26 | 12.00 | 0 | 200 | 0 | 0 | 0 |
| | 24.00 | 0 | 400 | 0 | 0 | 0 |
| 27 | 12.00 | 0 | 200 | 0 | 0 | 0 |
| | 24.00 | 0 | 400 | 0 | 0 | 0 |

| COMPOUND | CRW RATE PPM | CRW % | SAW SM & MA RATE PPM | SAW % | SM % | MA % |
|---|---|---|---|---|---|---|
| 28 | 12.00 | 0 | 200 | 100 | 0 | 0 |
| | 24.00 | 100 | 400 | 0 | 0 | 0 |
| 29 | 12.00 | 0 | 200 | 0 | 0 | 0 |
| | 24.00 | 0 | 400 | 0 | 0 | 0 |
| 30 | 12.00 | 0 | 200 | 0 | 100 | 100 |
| | 24.00 | 100 | 400 | 0 | 80 | 80 |
| 31 | 12.00 | 0 | 200 | 0 | 100 | 100 |
| | 24.00 | 0 | 400 | 0 | 100 | 20 |
| 32 | 12.00 | 0 | 200 | 100 | 0 | 0 |
| | 24.00 | 0 | 400 | 0 | 0 | 0 |
| 33 | 12.00 | 0 | 200 | 0 | 60 | 90 |
| | 24.00 | 0 | 400 | 0 | 0 | 0 |
| 34 | 12.00 | 0 | 200 | 0 | 0 | 0 |
| | 24.00 | 0 | 400 | 0 | 0 | 70 |
| 35 | 12.00 | 0 | 200 | 0 | 0 | 80 |
| | 24.00 | 80 | 400 | 0 | 0 | 80 |
| 36 | 12.00 | 0 | 200 | 0 | 0 | 100 |
| | 24.00 | 0 | 400 | 0 | 0 | 0 |
| 37 | 12.00 | 0 | 200 | 100 | 0 | 100 |
| | 24.00 | 60 | 400 | 40 | 0 | 0 |
| 38 | 12.00 | 0 | 200 | 0 | 30 | 80 |
| | 24.00 | 0 | 400 | 0 | 0 | 0 |
| 39 | 12.00 | 0 | 200 | 0 | 0 | 0 |
| | 24.00 | 0 | 400 | 0 | 0 | 0 |
| 40 | 12.00 | 0 | 200 | 0 | 30 | 20 |
| | 24.00 | 0 | 400 | 0 | 0 | 0 |

| COMPOUND | CRW RATE PPM | CRW % | SAW SM & MA RATE PPM | SAW % | SM % | MA % |
|---|---|---|---|---|---|---|
| 41 | 12.00 | 0 | 200 | 0 | 0 | 0 |
| | 24.00 | 0 | 400 | 0 | 0 | 0 |
| 42 | 12.00 | 0 | 200 | 0 | 0 | 0 |
| | 24.00 | 0 | 400 | 0 | 0 | 0 |
| 43 | 12.00 | 0 | 200 | 0 | 0 | 0 |
| | 24.00 | 0 | 400 | 0 | 0 | 0 |
| 44 | 12.00 | 0 | 200 | 0 | 0 | 0 |
| | 24.00 | 0 | 400 | 0 | 0 | 0 |
| 45 | 12.00 | 0 | 200 | 0 | 0 | 0 |
| | 24.00 | 0 | 400 | 0 | 0 | 0 |
| 46 | 12.00 | 0 | 200 | 100 | 0 | 0 |
| | 24.00 | 0 | 400 | 0 | 0 | 0 |
| 47 | 12.00 | 0 | 200 | 0 | 100 | 90 |
| | 24.00 | 0 | 400 | 0 | 0 | 0 |
| 48 | 12.00 | 40 | 200 | 0 | 0 | 0 |
| | 24.00 | 0 | 400 | 0 | 0 | 80 |
| 49 | 12.00 | 0 | 200 | 0 | 100 | 100 |
| | 24.00 | 0 | 400 | 0 | 100 | 90 |
| 50 | 12.00 | 0 | 200 | 0 | 60 | 90 |
| | 24.00 | 0 | 400 | 0 | 90 | 100 |
| 51 | 12.00 | 0 | 200 | 0 | 0 | 0 |
| | 24.00 | 0 | 400 | 0 | 100 | 100 |
| 52 | 12.00 | 0 | 200 | 100 | 100 | 100 |
| | 24.00 | 100 | 400 | 0 | 100 | 100 |
| 53 | 24.00 | 100 | 400 | 20 | 90 | 90 |

| COMPOUND | CRW RATE PPM | CRW % | SAW SM & MA RATE PPM | SAW % | SM % | MA % |
|---|---|---|---|---|---|---|
| 54 | 24.00 | 0 | 400 | 0 | 0 | 90 |
| 55 | 24.00 | 0 | 400 | 40 | 100 | 100 |
| 57 | 12.00 | 100 | 200 | 0 | 0 | 100 |
| | 24.00 | 100 | 400 | 100 | 0 | 90 |
| 58 | 12.00 | 100 | 200 | 0 | 90 | 100 |
| | 24.00 | 100 | 400 | 80 | 100 | 100 |
| 59 | 12.00 | 0 | 200 | 100 | 0 | 0 |
| | 24.00 | 0 | 400 | 0 | 0 | 0 |
| 60 | 24.00 | 0 | 400 | 0 | 0 | 90 |
| 61 | 12.00 | 0 | 200 | 0 | 100 | 90 |
| | 24.00 | 0 | 400 | 0 | 0 | 0 |
| 62 | 12.00 | 0 | 200 | 0 | 0 | 0 |
| | 24.00 | 0 | 400 | 0 | 0 | 0 |
| 63 | 12.00 | 0 | 200 | 0 | 0 | 0 |
| | 24.00 | 0 | 400 | 0 | 0 | 0 |
| 64 | 12.00 | 0 | 200 | 0 | 0 | 0 |
| | 24.00 | 0 | 400 | 0 | 0 | 0 |
| 65 | 12.00 | 100 | 200 | 60 | 0 | 0 |
| | 24.00 | 100 | 400 | 60 | 0 | 0 |
| 66 | 12.00 | 0 | 200 | 0 | 0 | 0 |
| | 24.00 | 0 | 400 | 100 | 0 | 0 |
| 67 | 24.00 | 100 | 400 | 0 | 80 | 100 |
| 73 | 12.00 | 0 | 200 | 0 | 0 | 0 |
| | 24.00 | 0 | 400 | 0 | 0 | 0 |

| COMPOUND | CRW RATE PPM | CRW % | SAW SM & MA RATE PPM | SAW % | SM % | MA % |
|---|---|---|---|---|---|---|
| 74 | 12.00 | 0 | 200 | 0 | 0 | 0 |
| | 24.00 | 0 | 400 | 0 | 0 | 0 |
| 75 | 12.00 | 0 | 200 | 0 | 0 | 0 |
| | 24.00 | 0 | 400 | 0 | 0 | 0 |
| 76 | 12.00 | 0 | 200 | 0 | 0 | 0 |
| | 24.00 | 0 | 400 | 0 | 0 | 0 |
| 77 | 12.00 | 0 | 200 | 0 | 100 | 100 |
| | 24.00 | 100 | 400 | 0 | 100 | 100 |
| 78 | 12.00 | 0 | 200 | 0 | 0 | 0 |
| | 24.00 | 0 | 400 | 0 | 0 | 0 |
| 79 | 12.00 | 0 | 200 | 0 | 0 | 0 |

## Compositions

The compounds of this invention are applied in the form of compositions which are important embodiments of the invention, and which comprise a compound of this invention and a phtologically-acceptable inert carrier. The compositions are either concentrated formulations which are dispersed in water for application, or are dust or granular formulations which are applied without further treatment. The compositions are prepared according to procedures and formulae which are conventional in the agricultural chemical art, but which are novel and important because of the presence therein of the compounds of this invention. Some description of the formulation of the compositions will be given, however, to assure that agricultural chemists can readily prepare any desired composition.

The dispersions in which the compounds are applied are most often aqueous suspensions or emulsions prepared from concentrated formulations of the compounds. Such water-soluble, water-suspendable or emulsifiable formulations are either solids usually known as wettable powders, or liquids usually known as emulsifiable concentrates or aqueous suspensions. Wettable powders, which may be compacted to form water dispersible granules, comprise an intimate mixture of the active compound, an inert carrier and surfactants. The concentration of the active compound is usually from about 10% to about 90% by weight. The inert carrier is usually chosen from among the attapulgite clays, the montmorillonite clays, the diatomaceous earths, or the purified silicates. Effective surfactants, comprising from about 0.5% to about 10% of the wettable powder, are found among the sulfonated lignins, the condensed naphthalenesulfonates, the naphthalenesulfonates, the alkylbenzenesulfonates, the alkyl sulfates, and nonionic surfactants such as ethylene oxide adducts of alkyl phenols.

Emulsifiable concentrates of the compounds comprise a conveninet concentration of a compound, such as from about 50 to about 500 grams per liter of liquid, equivalent to about 10% to about 50%, dissolved in an inert carrier which is either a water miscible solvent or a mixture of water-immiscible organic solvent and emulsifiers. Useful organic solvents include aromatics, especially the xylenes, and the petroleum fractions, especially the high-boiling naphthalenic and olefinic portions of petroleum such as heavy aromatic naphtha. Other organic solvents may also be used, such as the terpenic solvents including rosin derivatives, aliphatic ketones such as cyclohexanone, and complex alcohols such as 2-ethoxyethanol. Suitable emulsifiers for emulsifiable concentrates are chosen from conventional nonionic surfactants, such as those discussed above.

Aqueous suspensions comprise suspensions of water-insoluble compounds of this invention, dispersed in an aqueous vehicle at a concentration in the range from about 5% to about 50% by weight. Suspensions are prepared by finely grinding the compound, and vigorously mixing it into a vehicle comprised of water and surfactants chosen from the same types discussed above. Inert ingredients, such as inorganic salts and synthetic or natural gums, maw also be added, to increase the density and viscosity of the aqueous vehicle. It is often most effective to grind and mix the compound at the same time by preparing the aqueous mixture, and homogenizing it in an implement such as a sand mill, ball mill, or pistontype homogenizer.

The compounds may also be applied as granular compositions, which are particularly useful for application to the soil. Granular compositions usually contain from about 0.5% to about 10% by weight of the compound, dispersed in an inert carrier which consists entirely or in large part of clay or a similar inexpensive substance. Such compositions are usually prepared by dissolving the compound in a suitable solvent, and applying it to a granular carrier which has been pre-formed to the appropriate particle size, in the range of from about 0.5 to 3 mm. Such compositions may also be formulated by making a dough or paste of the carrier and compound, and crushing and drying to obtain the desired granular particle size.

Dusts containing the compounds are prepared simply by intimately mixing the compound in powdered form with a suitable dusty agricultural carrier, such as kaolin clay, ground volcanic rock and the like. Dusts can suitably contain from about 1% to about 10% of the compound.

It is equally practical, when desirable for any reason, to apply the compound in the form of a solution in an appropriate organic solvent, usually a bland petroleum oil, such as the spray oils, which are widely used in agricultural chemistry.

Insecticides and miticides are generally applied in the form of a dispersion of the active ingredient in a liquid carrier. It is conventional to refer to application rates in terms of the concentration of active ingredient in the carrier. The most widely used carrier is water.

The compounds of the invention can also be applied in the form of an aerosol composition. In such compositions the active compound is dissolved or dispersed in an inert carrier, which is a pressure-generating propellant mixture. The aerosol composition is package in a container from which the mixture is dispensed through an atomizing valve. Propellant mixtures comprise either low-boiling halocarbons, which may be mixed with organic solvents, or aqueous suspensions pressurized with inert gases or gaseous hydrocarbons.

The actual amount of compound to be applied to loci of insects and mites is not critical and can readily be determined by those skilled in the art in view of the examples above. In general, concentrations of from 10 ppm to 5000 ppm of compound are expected to provide good control. With many of the compounds, concentrations of from 100 to 1500 ppm will suffice. For field crops, such as soybeans and cotton, a suitable application rate for the compounds is about 0.5 to 1.5 lb/A, typically applied in 50 gal/A of spray formulation containing 1200 to 3600 ppm of compound. For citrus crops, a suitable application rate is from about 100 to 1500 gal/A spray formulation, which is a rate of 100 to 1000 ppm.

The locus to which a compound is applied can be any locus inhabited by an insect or arachnid, for example, vegetable crops, fruit and nut trees, grape vines, and ornamental plants. Inasmuch as many mite species are specific to a particular host, the foregoing list of mite species provides exemplification of the wide range of settings in which the present compounds can be used.

Because of the unique ability of mite eggs to resist toxicant action, repeated applications may be desirable to control newly emerged larvae, as is true of other known acaricides.

The following formulations of compounds of the invention are typical of compositions useful in the practice of the present invention.

### A. 0.75 Emulsifiable Concentrate

| | |
|---|---|
| N-[2-[4-(1,1,2,2-tetrafluoro-ethoxy)-phenyl]ethyl]thieno[2,3-d]pyrimidin-4-amine | 9.38% |
| "TOXIMUL D" (nonionic/anionic surfactant blend) | 2.50% |
| "TOXIMUL H" (nonionic/anionic surfactant blend) | 2.50% |
| "EXXON 200" (naphthalenic solvent) | 85.62% |

### B. 1.5 Emulsifiable Concentrate

| | |
|---|---|
| N-[2-[4-(1,1,2,2-tetrafluoro-ethoxy)phenyl]ethyl]thieno[3,2-d]-pyrimidin-4-amine | 18.50% |
| "TOXIMUL D" | 2.50% |
| "TOXIMUL H" | 2.50% |
| "EXXON 200" | 76.50% |

### C. 1.0 Emulsifiable Concentrate

| | |
|---|---|
| 5-methyl-N-[2-(2-naphthyl)ethyl]-thieno[2,3-d]pyrimidin-4-amine | 12.50% |
| N-methylpyrrolidone | 25.00% |
| "TOXIMUL D" | 2.50% |
| "TOXIMUL H" | 2.50% |
| "EXXON 200" | 57.50% |

D. 1.0 Aqueous Suspension

| | |
|---|---|
| N-[2-[4-(1,1,2,2-tetrafluoro-ethoxy)-phenyl]ethyl]thieno[2,3-d]pyrimidin-4-amine | 12.00% |
| "PLURONIC P-103" (block copolymer of propylene oxide and ethylene oxide, surfactant) | 1.50% |
| "PROXEL GXL" (biocide/preservative) | .05% |
| "AF-100" (silicon based antifoam agent) | .20% |
| "REAX 88B" (lignosulfonate dispersing agent) | 1.00% |
| propylene glycol | 10.00% |
| veegum | .75% |
| xanthan | .25% |
| water | 74.25% |

E. 1.0 Aqueous Suspension

| | |
|---|---|
| 5-methyl-N-[2-[4-(1,1,2,2-tetrafluoroethoxy)phenyl]ethyl]thieno[2,3-d]-pyrimidin-4-amine | 12.50% |
| "MAKON 10" (10 moles ethyleneoxide nonylphenol surfactant) | 1.00% |
| "ZEOSYL 200" (silica) | 1.00% |
| "AF-100" | 0.20% |
| "AGRIWET FR" (surfactant) | 3.00% |
| 2% xanthan hydrate | 10.00% |
| water | 72.30% |

### F. 1.0 Aqueous Suspension

| | |
|---|---|
| N-[2-[4-(2,2,2-trifluoroethoxy)phenyl]-ethyl]thieno[2,3-d]pyrimidin-4-amine | 12.50% |
| "MAKON 10" | 1.50% |
| "ZEOSYL 200" (silica) | 1.00% |
| "AF-100" | 0.20% |
| "POLYFON H" (lignosulfonate dispersing agent) | 0.20% |
| 2% xanthan hydrate | 10.00% |
| water | 74.60% |

### G. Wettable Powder

| | |
|---|---|
| N-[2-[4-(1,1,2,2-tetrafluoro-ethoxy)-phenyl]ethyl]thieno[2,3-d]pyrimidin-4-amine | 25.80% |
| "POLYFON H" | 3.50% |
| "SELLOGEN HR" | 5.00% |
| "STEPANOL ME DRY" | 1.00% |
| gum arabic | 0.50% |
| "HISIL 233" | 2.50% |
| Barden clay | 61.70% |

H. 1.0 Aqueous Suspension

| | |
|---|---|
| N-[2-[4-(t-butyl)phenyl]ethyl]-thieno[3,2-d]pyrimidin-4-amine | 12.40% |
| "TERGITOL 158-7" | 5.00% |
| "ZEOSYL 200" | 1.00% |
| "AF-100" | 0.20% |
| "POLYFON H" | 0.50% |
| 2% xanthan solution | 10.00% |
| tap water | 70.90% |

I. 1.0 Emulsifiable Concentrate

| | |
|---|---|
| N-[2-[4-(2,2,2-trifluoroethoxy)phenyl]-ethyl]thieno[2,3-d]pyrimidin-4-amine | 12.40% |
| "TOXIMUL D" | 2.50% |
| "TOXIMUL H" | 2.50% |
| "EXXON 200" | 82.60% |

J. Wettable Powder

| | |
|---|---|
| N-[2-[4-(1,1,2,2-tetrafluoroethoxy)-phenyl]ethyl]thieno[3,2-d]pyrimidin-4-amine | 25.80% |
| "SELLOGEN HR" | 5.00% |
| "POLYFON H" | 4.00% |
| "STEPANOL ME DRY" | 2.00% |
| "HISIL 233" | 3.00% |
| Barden clay | 60.20% |

K. 0.5 Emulsifiable Concentrate

| | |
|---|---|
| N-[2-(2-naphthyl)ethyl]thieno[2,3-d]-pyrimidin-4-amine | 6.19% |
| "TOXIMUL H" | 3.60% |
| "TOXIMUL D" | 0.40% |
| "EXXON 200" | 89.81% |

## Claims

1. A compound of formula (1)

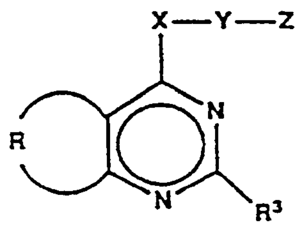

(1)

wherein

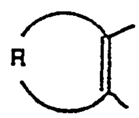 is 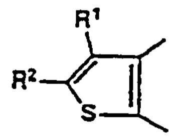, 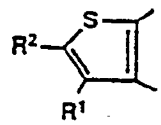, or 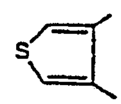.

$R^1$ is H, $C_1$1-$C_4$ alkyl, or phenyl optionally substituted with halo, ($C_1$-$C_4$) alkyl, ($C_1$-$C_4$) alkoxy, halo ($C_1$-$C_4$) alkyl, or halo ($C_1$-$C_4$) alkoxy;

$R^2$ is H, ($C_1$-$C_4$) alkyl, nitro, halo; or

$R^1$ and $R^2$ combine to form $-(CH_2)_4-$;

$R^3$ is H, ($C_1$-$C_4$) alkyl, halo ($C_1$-$C_4$) alkyl, phenyl, or substituted phenyl;

-XYZ together form 1H-1,2,4-triazol-1-yl; or

X is O, S, $NR^4$, or $CR^5R^6$, where $R^4$ is H, ($C_1$-$C_4$) alkyl, or ($C_1$-$C_4$) acyl, and $R^5$ and $R^6$ are independently H, ($C_1$-$C_4$) alkyl, ($C_2$-$C_4$) alkenyl or -alkynyl, CN, cr OH, or $R^5$ and $R^6$ combine to form a carbocyclic ring containing four to six carbon atoms; and

-YZ together form a $C_5$-$C_{12}$ saturated or unsaturated hydrocarbon chain, straight chain or branched; optionally including a hetero atom selected from O, $NR^4$, S, SO, $SO_2$, or $SiR^7R^8$, where $R^7$ and $R^8$ are independently ($C_1$-$C_4$) alkyl, ($C_3$-$C_4$) branched alkyl, phenyl, or substituted phenyl, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl; or

Y is a bond or a bivalent hydrocarbon radical one to six carbon atoms long, optionally including a carbocyclic ring, and optionally including a hetero atom selected from O, $NR^4$, S, SO, $SO_2$, or $SiR^7R^8$, where $R^4$, $R^7$, and $R^8$ are as defined above, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl; and

Z is

(a) ($C_3$-$C_8$) cycloalkyl or cycloalkenyl, optionally substituted with ($C_1$-$C_4$) alkyl, ($C_1$-$C_4$) alkoxy, halo ($C_1$-$C_4$) alkyl, halo ($C_1$-$C_4$) alkoxy, halo, hydroxy, or ($C_1$-$C_4$) acyl;

(b) a group of the formula (2)

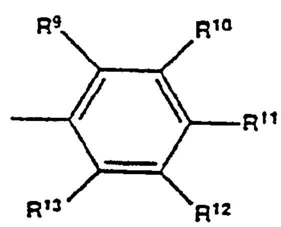

(2)

where

$R^9$ to $R^{13}$ are independently

H,

halo,
I,
($C_3$-$C_8$) cycloalkyl or -cycloalkenyl,
phenoxy, or
substituted phenoxy,
phenylthio, or substituted phenylthio,
phenyl, or substituted phenyl,
$NO_2$,
acetoxy,
OH,
CN,
$SiR^7R^8R^{14}$ or $OSiR^7R^8R^{14}$, where $R^7$, and $R^8$ are as defined above and $R^{14}$ is ($C_1$-$C_4$) alkyl, ($C_3$-$C_4$) branched alkyl, phenyl, or substituted phenyl,
$NR^{15}R^{16}$, where $R^{15}$ and $R^{16}$ are independently H, ($C_1$-$C_4$) alkyl, or ($C_1$-$C_4$) acyl,
$S(O)R^{17}$, or $SO_2R^{17}$, where $R^{17}$ is ($C_1$-$C_{10}$) akyl, phenyl, or substituted phenyl;
a $C_1$-$C_{12}$ saturated or unsaturated hydrocarbon chain, straight chain or branched optionally including a hetero atom selected from O, S, SO, $SO_2$, $NR^4$, or $SiR^7R^8$, where $R^4$, $R^7$ and $R^8$ are as defined above, and optionally substituted with halo, halo ($C_1$-$C_4$) alkoxy, hydroxy, ($C_3$-$C_8$) cycloalkyl or cycloalkenyl, ($C_1$-$C_4$) acyl, phenoxy, substituted phenoxy, phenyl, substituted phenyl, phenylthio, or substituted phenylthio;
($C_1$-$C_7$) alkoxy optionally substituted with halo, phenyl, substituted phenyl, ($C_3$-$C_8$) cycloalkyl or cycloalkenyl, phenoxy, or substituted phenoxy;
($C_1$-$C_7$) alkylthio optionally substituted with halo, phenyl, substituted phenyl, ($C_3$-$C_8$) cycloalkyl or cycloalkenyl, phenoxy or substituted phenoxy; or
$R^{10}$ and $R^{11}$ combine to form -O-$CF_2$-O-;
(c) a furyl group of formula (3)

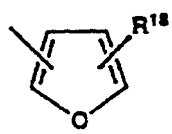 (3)

where $R^{18}$ is H, halo, halomethyl, CN, $NO_2$, ($C_1$-$C_4$) alkyl, ($C_2$-$C_4$) branched alkyl, phenyl, ($C_1$-$C_4$) alkoxy;
(d) a thienyl group of the formula (4)

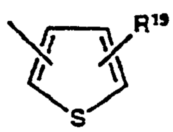 (4)

where $R^{19}$ is $R^{18}$ as defined in paragraph (c) or thienyl;
(e) a group of formula (5) or (6)

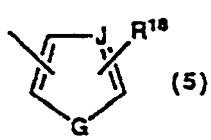 (5) –

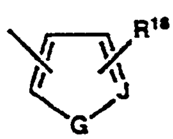 (6)

where $R^{18}$ is as defined in paragraph (c), J is N or CH, and G is O, $NR^{20}$, or S, provided that if J is not N then G is $NR^{20}$, where $R^{20}$ is H, ($C_1$-$C_4$) alkyl, ($C_1$-$C_4$) acyl, phenylsulfonyl, or substituted phenylsulfonyl;
(f) a group selected from ,
optionally substituted naphthyl, dihydronaphthyl, tetrahydronaphthyl, and decahydronanhthyl;
optionally substituted pyridyl;
optionally substituted indolyl;
1,3-benzodioxolyl;

11. The use of a compound as claimed in any one of claims 1 to 8 as a fungicide, an insecticide, or a miticide.

12. A method for the control or prevention of fungal, insect, or mite infestation, which method comprises applying to the infestation, or to a locus in which fungal, insect or mite infestation is to be prevented, a compound as claimed in any one of claims 1 to 8, or a composition as claimed in claim 10.

13. A method for the preparation of a compound of formula (1) as defined in claim 1, which method comprises

(a) condensing a compound of formula (23)

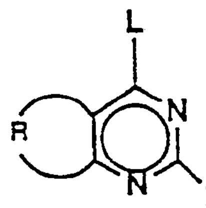

(23)

wherein R and $R^3$ are as defined in claim 1, and L is a leaving group, with an alcohol of the formula (8)

OH-Y-Z (8),

wherein Y and Z are as defined in claim 1;

(b) condensing a compound of formula (23) as defined above with an amine of the formula (9)

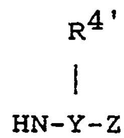

(9)

where $R^{4'}$ is H or ($C_1$-$C_4$) alkyl, and Y and Z are as previously defined, to provide a compound of formula (1) where X is $NR^{4'}$; or

(c) acylating a compound of formula (1) wherein X is NH to provide a compound of formula (1) wherein X is $NR^4$ and $R^4$ is acyl; or

(d) reacting a compound of formula (23) wherein L is H with a Grignard reagent of formula

$Z\text{-}Y\text{-}CR^5R^6\text{-}MgX'$

where X' is halo and Z and Y are as defined above, or with a lithium-containing reagent of formula

$Z\text{-}Y\text{-}CR^5R^6\text{-}Li$

to provide a 3,4-dihydro thienopyrimidine, which is then oxidized to produce a compound of formula (1) wherein X is $CR^5R^6$; or

(e) hydrolyzing and decarboxylating a 5-substituted-5-(4-(thienopyrimidinyl)barbituric acid of the formula

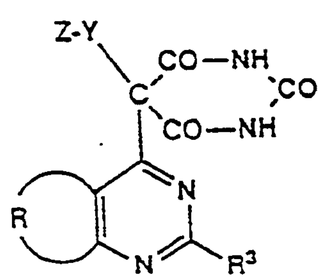

wherein R, $R^3$, $R^5$, $R^6$, Y, and Z are as defined above, to provide a compound of formula (1) wherein X is $CH_2$; or

(f) heating a compound of the formula (7)

2,6-dimethyl-4-morpholinyl; and
1-adamantyl;

or an acid addition salt of a compound of formula (1); provided that:

(1) it the compound of formula (1) is a thieno[2,3-d]pyrimidin-4-amine, then Y does not include a heteroatom and is at least two carbon atoms long, and Z is 2-naghthyl or a pentyl group of formula (2) wherein at least one of $R^9$ to $R^{13}$ is phenyl, substituted phenyl, phenoxy, substituted phenoxy, phenylthio, substituted phenylthio, ($C_3$-$C_4$) branched alkyl, ($C_1$-$C_4$) alkoxy, halo, halo ($C_1$-$C_4$) alkoxy, or halo ($C_1$-$C_4$) alkyl;

(2) if the compound of formula (1) is a thieno[2,3-d]pyrimidine wherein X is 0, then YZ is neither unsubstituted phenyl nor benzyl; and

( 3 ) if the compound of formula (1) is a 7-methylthieno[3,2-d]pyrimidine, then -XYZ is not N-[2-(2-pyridyl)ethyl]amino, and X-Y does not include the group -NH-N=CH-.

2. A compound of claim 1 that is a thieno[2,3-d]pyrimidine.

3. A compound of claim 1 or claim 2 wherein $R^1$ is H or methyl and $R^2$ is H.

4. A compound of any one of the preceding claims, wherein Y is $-CH_2-CH_2-$.

5. A compound of any one of the preceding claims, wherein Z is 2-naphthyl or a group of formula (2).

6. A compound of any one of the preceding claims, wherein X is NH.

7. A compound of any one of claims 1 to 5 wherein X is O.

8. A compound of formula (7)

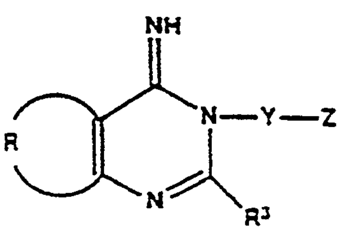

(7)

wherein R, $R^3$ and Z are as defined in claim 1 and

Y is a bivalent hydrocarbon radical two to six carbon atoms long, optionally including a carbocyclic ring, and optionally including a hetero atom selected from O, $NR^4$, S, SO, $SO_2$, or $SiR^7R^8$, where $R^4$, $R^7$, and $R^8$ are as defined above, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl

9. A compound of the formula (8)

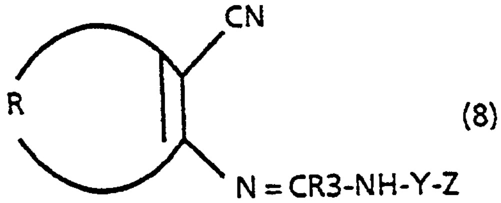

(8)

wherein R, $R^3$ and Z are defined in Claim 1 and Y is as defined in Claim 8

10. A fungicide, insecticide or miticide composition which comprises a compound as claimed in any one of claims 1 to 8 in combination with a phytologically-acceptable carrier.

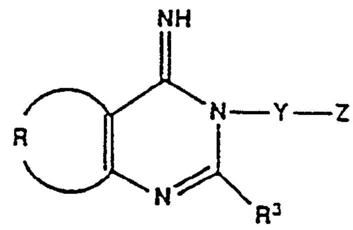

(7)

optionally in the presence of base to provide a compound of the formula (1d)

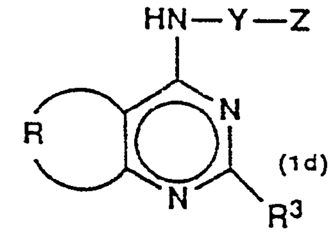

(1d)

**Claims for the following Contracting States: ES**

1. The use as a fungicide, insecticide or miticide of a compound of formula (1)

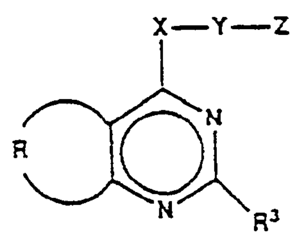

(1)

wherein

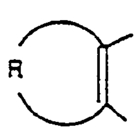 is 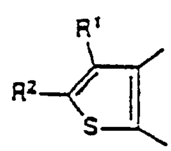 , 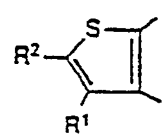 , or 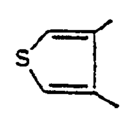

$R^1$ is H, $C_1$-$C_4$ alkyl, or phenyl optionally substituted with halo, ($C_1$-$C_4$) alkyl, ($C_1$-$C_4$) alkoxy, halo ($C_1$-$C_4$) alkyl, or halo ($C_1$-$C_4$) alkoxy;

$R^2$ is H, ($C_1$-$C_4$) alkyl, nitro, halo; or

$R^1$ and $R^2$ combine to form $-(CH_2)_4-$;

$R^3$ is H, ($C_1$-$C_4$) alkyl, halo ($C_1$-$C_4$) alkyl, phenyl, or substituted phenyl;

-XYZ together form 1H-1,2,4-triazol-1-yl; or

X is O, S, $NR^4$, or $CR^5R^6$, where $R^4$ is H, ($C_1$-$C_4$) alkyl, or ($C_1$-$C_4$) acyl, and $R^5$ and $R^6$ are independently H, ($C_1$-$C_4$) alkyl, ($C_2$-$C_4$) alkenyl or -alkynyl, CN, or OH, or $R^5$ and $R^6$ combine to form a carbocyclic ring containing four to six carbon atoms; and

-YZ together form a $C_5$-$C_{12}$ saturated or unsaturated hydrocarbon chain, straight chain or branched; optionally including a hetero atom selected from O, $NR^4$, S, SO, $SO_2$, or $SiR^7R^8$, where $R^7$ and $R^8$ are independently ($C_1$-$C_4$) alkyl, ($C_3$-$C_4$) branched alkyl, phenyl, or substituted phenyl, and optionally substituted with ($C_1$-$C_4$) alkyl, ($C_2$-$C_4$) alkenyl or -alkynyl, branched ($C_3$-$C_7$) alkyl, ($C_2$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl; or

Y is a bond or a bivalent hydrocarbon radical one to six carbon atoms long, optionally including a carbocyclic ring, and optionally including a hetero atom selected from O, $NR^4$, S, SO, $SO_2$, or $SiR^7R^8$, where

$R^4$, $R^7$, and $R^8$ are as defined above, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl; and

Z is

(a) ($C_3$-$C_8$) cycloalkyl or cycloalkenyl, optionally substituted with ($C_1$-$C_4$) alkyl, ($C_1$-$C_4$) alkoxy, halo ($C_1$-$C_4$) alkyl, halo ($C_1$-$C_4$) alkoxy, halo, hydroxy or ($C_1$-$C_4$) acyl;

(b) a group of the formula (2)

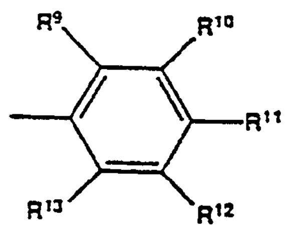

(2)

where

$R^9$ to $R^{13}$ are independently

H,

halo,

I,

($C_3$-$C_8$) cycloalkyl or -cycloalkenyl,

phenox, or

substituted phenoxy,

phenylthio, or substituted phenylthio,

phenyl, or substituted phenyl,

$NO_2$,

acetoxy,

OH,

CN,

$SiR^7R^8R^{14}$ or $OSiR^7R^8R^{14}$, where $R^7$, and $R^8$ are as defined above and $R^{14}$ is ($C_1$-$C_4$) alkyl, ($C_3$-$C_4$) branched alkyl, phenyl, or substituted phenyl,

$NR^{15}R^{16}$, where $R^{15}$ and $R^{16}$ are independently H, ($C_1$-$C_4$) alkyl, or ($C_1$-$C_4$) acyl,

$S(O)R^{17}$, or $SO_2R^{17}$, where $R^{17}$ is ($C_1$-$C_{10}$) alkyl, phenyl, or substituted phenyl;

a $C_1$-$C_{12}$ saturated or unsaturated hydrocarbon chain, straight chain or branched optionally including a hetero atom selected from O, S, SO, $SO_2$, $NR^4$, or $Sir^7R^8$, where $R^4$, $R^7$ and $R^8$ are as defined above, and optionally substituted with halo, halo ($C_1$-$C_4$) alkoxy, hydroxy, ($C_3$-$C_8$) cycloalkyl or cycloalkenyl, ($C_1$-$C_4$) acyl, phenoxy, substituted phenoxy, phenyl, substituted phenyl, phenylthio, or substituted phenylthio;

($C_1$-$C_7$) alkoxy optionally substituted with halo, phenyl, substituted phenyl, ($C_3$-$C_8$) cycloalkyl or cycloalkenyl, phenoxy, or substituted phenoxy;

($C_1$-$C_7$) alkylthio optionally substituted with halo, phenyl, substituted phenyl, ($C_3$-$C_8$) cycloalkyl or cycloalkenyl, phenoxy or substituted phenoxy; or

$R^{10}$ and $R^{11}$ combine to form $-O-CF_2-O-$;

(c) a furyl group of formula (3)

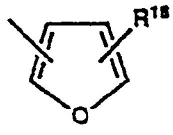

(3)

where $R^{18}$ is H, halo, halomethyl, CN, $NO_2$, ($C_1$-$C_4$) alkyl, ($C_3$-$C_4$) branched alkyl, phenyl, ($C_1$-$C_4$) alkoxy;

(d) a thienyl group of the formula (4)

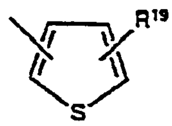

(4)

where $R^{19}$ is $R^{18}$ as defined in paragraph (c) or thienyl;

(e) a group of formula (5) or (6)

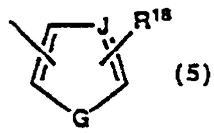

(5)

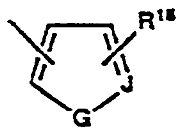

(6)

where $R^{18}$ is as defined in paragraph (c), J is N or CH, and G is O, $NR^{20}$, or S, provided that if J is not N then G is $NR^{20}$, where $R^{20}$ is H, $(C_1-C_4)$ alkyl, $(C_1-C_4)$ acyl, phenylsulfonyl, or substituted phenylsulfonyl;

(f) a group selected from

optionally substituted naphthyl, dihydronaphthyl, tetrahydronaphthyl, and decahydronaphthyl;

optionally substituted pyridyl;

optionally substituted indolyl;

1,3-benzodioxolyl;

2,6-dimethyl-4-morpholinyl; and

1-adamantyl;

or an acid addition salt of a compound of formula (1); provided that:

(1) if the compound of formula (1) is a thieno[2,3-d]pyrimidin-4-amine, then Y does not include a heteroatom and is at least two carbon atoms long, and Z is 2-naphthyl or a pentyl group of formula (2) wherein at least one of $R^9$ to $R^{13}$ is phenyl, substituted phenyl, phenoxy, substituted phenoxy, phenylthio, substituted phenylthio, $(C_3-C_4)$ branched alkyl, $(C_1-C_4)$ alkoxy, halo, halo $(C_1-C_4)$ alkoxy, or halo $(C_1-C_4)$ alkyl;

(2) if the compound of formula (1) is a thieno[2,3-d]pyrimidime wherein X is O, then YZ is neither unsubstituted phenyl nor benzyl; and

(3) if the compound of formula (1) is a 7-methylthieno[3,2-d]pyrimididine, then -XYZ is not N-[2-(2-pyridyl)ethyl]amino, and X-Y does not include the group -NH-N=CH-.

2. The use of claim 1, wherein the said compound is a thieno[2,3-d]pyrimidine.

3. The use of claim 1 or claim 2 wherein $R^1$ is H or methyl and $R^2$ is H.

4. The use of any one of the preceding claims, wherein Y is $-CH_2-CH_2-$.

5. The use of any one of the preceding claims, wherein Z is 2-naphthyl or a group of formula (2).

6. The use of any one of the preceding claims, wherein X is NH.

7. The use of any one of claims 1 to 5 wherein X is O.

8. The use of a compound of formula (7)

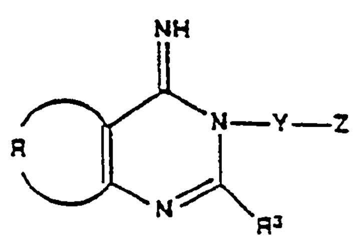

(7)

wherein R, $R^3$ and Z are as defined in claim 1 and

Y is a bivalent hydrocarbon radical two to six carbon atoms long, optionally including a carbocyclic ring, and optionally including a hetero atom selected from O, $NR^4$, S, SO, $SO_2$, or $SiR^7R^8$, where $R^4$, $R^7$, and $R^8$ are as defined above, and optionally substituted with ($C_1$-$C_4$) alkyl, ($C_2$-$C_4$) alkenyl or -alkynyl, branched ($C_3$-$C_7$) alkyl, ($C_3$-$C_7$) cycloalkyl or -cycloalkenyl, halo, halo ($C_1$-$C_4$) alkyl, halo ($C_1$-$C_4$) alkoxy, hydroxy, or ($C_1$-$C_4$) acyl, as a fungicide, insecticide, or miticide, or as an intermediate for the preparation of a compound as defined in claim 1.

**9.** The use of a compound of the formula (8)

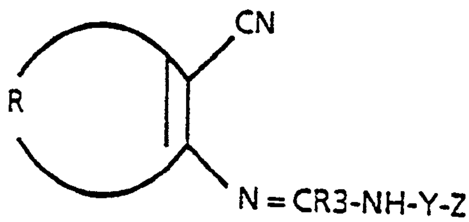

(8)

wherein R, $R^3$ and Z are defined in Claim 1 and Y is as defined in Claim 8 as an intermediate for the preparation of a compound as defined in any one of claims 1 to 8.

**10.** A fungicide insecticide or miticide composition which comprises a compound as defined in any one of claims 1 to 8 in combination with a phytologically-acceptable carrier.

**11.** A method for the control or prevention of fungal, insect, or mite infestation, which method comprises applying to the infestation, or to a locus in which fungal, insect or mite infestation is to be prevented, a compound as defined in any one of claims 1 to 8, or a composition as claimed in claim 10.

**12.** A method for the preparation of a compound of formula (1) as defined in claim 1, which method comprises (a) condensing a compound of formula (23)

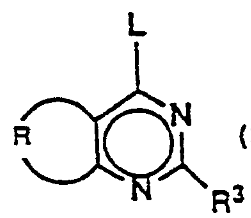

(23)

wherein R and $R^3$ are as defined in claim 1, and L is a leaving group, with an alcohol of the formula (8)

OH-Y-Z (8),

wherein Y and Z are as defined in claim 1;

(b) condensing a compound of formula (23) as defined above with an amine of the formula (9)

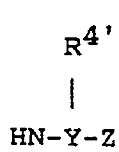

(9)

where $R^{4'}$ is H or ($C_1$-$C_4$) alkyl, and Y and Z are as previously defined, to provide a compound of formula (1) where X is $NR^{4'}$; or

(c) acylating a compound of formula (1) wherein X is NH to provide a compound of formula (1) wherein X is $NR^4$ and $R^4$ is acyl; or

(d) reacting a compound of formula (23) wherein L is H with a Grignard reagent of formula

$Z\text{-}Y\text{-}CR^5R^6\text{-}MgX'$

where X' is halo and Z and Y are as defined above, or with a lithium-containing reagent of formula

$$Z\text{-}Y\text{-}CR^5R^6\text{-}Li$$

to provide a 3,4-dihydro thienopyrimidine, which is then oxidized to produce a compound of formula (1) wherein X is $CR^5R^6$; or

(e) hydrolyzing and decarboxylating a 5-substituted-5-(4-(thienopyrimidinyl)barbituric acid of the formula

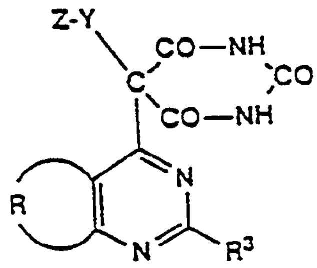

wherein R, $R^3$, $R^5$, $R^6$, Y, and Z are as defined above, to provide a compound of formula (1) wherein X is $CH_2$; or

(f) heating a compound of the formula (7)

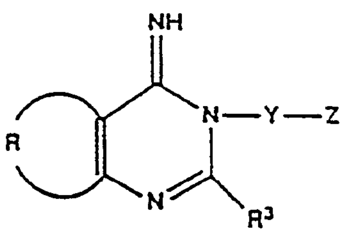

(7)

optionally in the presence of base to provide a compound of the formula (1d)

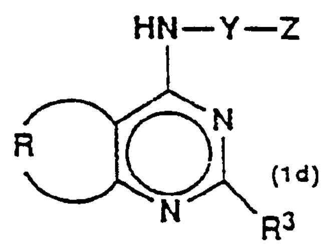

(1d)